# EUROPEAN PATENT SPECIFICATION

(11) **EP 1 439 221 B1**
(45) Date of publication and mention of the grant of the patent: **24.01.2007**
(21) Application number: 03028083.8
(22) Date of filing: 09.12.2003
(51) Int. Cl.: C12N 9/16, C07K 16/40, G01N 33/50, C12N 5/10

(54) **PDE core construct**
PDE Kern Konstrukt
Construction dérivée du noyau de PDE

(30) Priority: 17.12.2002 EP 02028057
(43) Date of publication of application: 21.07.2004
(73) Proprietor: F. HOFFMANN-LA ROCHE AG, 4070 Basel (CH)
(72) Inventor: Certa, Ulrich, 4123 Allschwil (CH); Fingerle, Juergen, 79400 Kandern (DE); Nelboeck-Hochstetter, Peter, 4054 Basel (CH); Schoenfeld, Hans-Joachim, 79115 Freiburg (DE)

(56) References cited:
- WO-A-02/22661
- WO-A-02/074992
- KOVALA TOM ET AL: "Recombinant expression of a type IV, cAMP-specific phosphodiesterase: Characterization and structure-function studies of deletion mutants" BIOCHEMISTRY, vol. 36, no. 10, 1997, pages 2968-2976, XP002278796 ISSN: 0006-2960
- KENAN YAEL ET AL: "Functions of the N-terminal region of cyclic nucleotide phosphodiesterase 3 (PDE 3) isoforms" JOURNAL OF BIOLOGICAL CHEMISTRY, vol. 275, no. 16, 21 April 2000 (2000-04-21), pages 12331-12338, XP002278797 ISSN: 0021-9258
- FISHER D A ET AL: "Isolation and characterization of PDE8A, a novel human cAMP-specific phosphodiesterase" BIOCHEMICAL AND BIOPHYSICAL RESEARCH COMMUNICATIONS, ACADEMIC PRESS INC. ORLANDO, FL, US, vol. 246, no. 3, 29 May 1998 (1998-05-29), pages 570-577, XP002091360 ISSN: 0006-291X
- BOLGER G B: "THE MULTIENZYME PDE4 CYCLIC ADENOSINE MONOPHOSPHATE-SPECIFIC PHOSPHODIESTERASE FAMILY: INTRACELLULAR TARGETING, REGULATION, AND SELECTIVE INHIBITION BY COMPOUNDS EXERTING ANTI-INFLAMMATORY AND ANTIDEPRESSANT ACTIONS" ADVANCES IN PHARMACOLOGY, SAN DIEGO, CA, US, vol. 44, 1998, pages 225-342, XP000906963
- GRAEME B BOLGER ET AL: "Characterization of five different proteins produced by alternatively spliced mRNAs from the human cAMP-specific phosphodiesterase PDE4D gene" BIOCHEMICAL JOURNAL, PORTLAND PRESS, LONDON, GB, vol. 328, 1997, pages 539-548, XP002150449 ISSN: 0264-6021

### SEQUENCE LISTING

<110> F. Hoffmann-La Roche AG
<120> Modified PDE4D polypeptides
<130> case 21507
<160> 6
<170> PatentIn version 3.1
<210> 1
<211> 673
<212> PRT
<213> Homo sapiens
<220>
<221> PDE4D3
<222> (1)..(673)
<223>
<220>
<221> LF1_splice_site
<222> (17)..(17)
<223>
<220>
<221> Ser579
<222> (579)..(579)
<223>
<220>
<221> Ser54
<222> (54)..(54)
<223>
<220>
<221> UCR1_site_-13
<222> (37)..(37)
<223>
<220>
<221> UCR1_start
<222> (50)..(50)
<223>
<400> 1
   Met Met His Val Asn Asn Phe Pro Phe Arg Arg His Ser Trp Ile Cys
   1 5 10 15
   Phe Asp Val Asp Asn Gly Thr Ser Ala Gly Arg Ser Pro Leu Asp Pro
   20 25 30
   Met Thr Ser Pro Gly Ser Gly Leu Ile Leu Gln Ala Asn Phe Val His
   35 40 45
   Ser Gln Arg Arg Glu Ser Phe Leu Tyr Arg Ser Asp Ser Asp Tyr Asp
   50 55 60
   Leu Ser Pro Lys Ser Met Ser Arg Asn Ser Ser Ile Ala Ser Asp Ile
   65 70 75 80
   His Gly Asp Asp Leu Ile Val Thr Pro Phe Ala Gln Val Leu Ala Ser
   85 90 95
   Leu Arg Thr Val Arg Asn Asn Phe Ala Ala Leu Thr Asn Leu Gln Asp
   100 105 110
   Arg Ala Pro Ser Lys Arg Ser Pro Met Cys Asn Gln Pro Ser Ile Asn
   115 120 125
   Lys Ala Thr Ile Thr Glu Glu Ala Tyr Gln Lys Leu Ala Ser Glu Thr
   130 135 140
   Leu Glu Glu Leu Asp Trp Cys Leu Asp Gln Leu Glu Thr Leu Gln Thr
   145 150 155 160
   Arg His Ser Val Ser Glu Met Ala Ser Asn Lys Phe Lys Arg Met Leu
   165 170 175
   Asn Arg Glu Leu Thr His Leu Ser Glu Met Ser Arg Ser Gly Asn Gln
   180 185 190
   Val Ser Glu Phe Ile Ser Asn Thr Phe Leu Asp Lys Gln His Glu Val
   195 200 205
   Glu Ile Pro Ser Pro Thr Gln Lys Glu Lys Glu Lys Lys Lys Arg Pro
   210 215 220
   Met Ser Gln Ile Ser Gly Val Lys Lys Leu Met His Ser Ser Ser Leu
   225 230 235 240
   Thr Asn Ser Ser Ile Pro Arg Phe Gly Val Lys Thr Glu Gln Glu Asp
   245 250 255
   Val Leu Ala Lys Glu Leu Glu Asp Val Asn Lys Trp Gly Leu His Val
   260 265 270
   Phe Arg Ile Ala Glu Leu Ser Gly Asn Arg Pro Leu Thr Val Ile Met
   275 280 285
   His Thr Ile Phe Gln Glu Arg Asp Leu Leu Lys Thr Phe Lys Ile Pro
   290 295 300
   Val Asp Thr Leu Ile Thr Tyr Leu Met Thr Leu Glu Asp His Tyr His
   305 310 315 320
   Ala Asp Val Ala Tyr His Asn Asn Ile His Ala Ala Asp Val Val Gln
   325 330 335
   Ser Thr His Val Leu Leu Ser Thr Pro Ala Leu Glu Ala Val Phe Thr
   340 345 350
   Asp Leu Glu Ile Leu Ala Ala Ile Phe Ala Ser Ala Ile His Asp Val
   355 360 365
   Asp His Pro Gly Val Ser Asn Gln Phe Leu Ile Asn Thr Asn Ser Glu
   370 375 380
   Leu Ala Leu Met Tyr Asn Asp Ser Ser Val Leu Glu Asn His His Leu
   385 390 395 400
   Ala Val Gly Phe Lys Leu Leu Gln Glu Glu Asn Cys Asp Ile Phe Gln
   405 410 415
   Asn Leu Thr Lys Lys Gln Arg Gln Ser Leu Arg Lys Met Val Ile Asp
   420 425 430
   Ile Val Leu Ala Thr Asp Met Ser Lys His Met Asn Leu Leu Ala Asp
   435 440 445
   Leu Lys Thr Met Val Glu Thr Lys Lys Val Thr Ser Ser Gly Val Leu
   450 455 460
   Leu Leu Asp Asn Tyr Ser Asp Arg Ile Gln Val Leu Gln Asn Met Val
   465 470 475 480
   His Cys Ala Asp Leu Ser Asn Pro Thr Lys Pro Leu Gln Leu Tyr Arg
   485 490 495
   Gln Trp Thr Asp Arg Ile Met Glu Glu Phe Phe Arg Gln Gly Asp Arg
   500 505 510
   Glu Arg Glu Arg Gly Met Glu Ile Ser Pro Met Cys Asp Lys His Asn
   515 520 525
   Ala Ser Val Glu Lys Ser Gln Val Gly Phe Ile Asp Tyr Ile Val His
   530 535 540
   Pro Leu Trp Glu Thr Trp Ala Asp Leu Val His Pro Asp Ala Gln Asp
   545 550 555 560
   Ile Leu Asp Thr Leu Glu Asp Asn Arg Glu Trp Tyr Gln Ser Thr Ile
   565 570 575
   Pro Gln Ser Pro Ser Pro Ala Pro Asp Asp Pro Glu Glu Gly Arg Gln
   580 585 590
   Gly Gln Thr Glu Lys Phe Gln Phe Glu Leu Thr Leu Glu Glu Asp Gly
   595 600 605
   Glu Ser Asp Thr Glu Lys Asp Ser Gly Ser Gln Val Glu Glu Asp Thr
   610 615 620
   Ser Cys Ser Asp Ser Lys Thr Leu Cys Thr Gln Asp Ser Glu Ser Thr
   625 630 635 640
   Glu Ile Pro Leu Asp Glu Gln Val Glu Glu Glu Ala Val Gly Glu Glu
   645 650 655
   Glu Glu Ser Gln Pro Glu Ala Cys Val Ile Asp Asp Arg Ser Pro Asp
   660 665 670
   Thr
<210> 2
<211> 664
<212> PRT
<213> Homo sapiens
<220>
<221> PDE4D_core
<222> (1)..(664)
<223>
<220>
<221> LF1_splice_site
<222> (2)..(2)
<223>
<220>
<221> UCR1_site
<222> (35)..(35)
<223>
<220>
<221> UCR1_site_-13
<222> (22)..(22)
<223>
<220>
<221> SER54
<222> (39)..(39)
<223>
<220>
<221> SER579
<222> (564)..(564)
<223>
<400> 2
   Met Phe Asp Val Asp Asn Gly Thr Ser Ala Gly Arg Ser Pro Leu Asp
   1 5 10 15
   Pro Met Thr Ser Pro Gly Ser Gly Leu Ile Leu Gln Ala Asn Phe Val
   20 25 30
   His Ser Gln Arg Arg Glu Ser Phe Leu Tyr Arg Ser Asp Ser Asp Tyr
   35 40 45
   Asp Leu Ser Pro Lys Ser Met Ser Arg Asn Ser Ser Ile Ala Ser Asp
   50 55 60
   Ile His Gly Asp Asp Leu Ile Val Thr Pro Phe Ala Gln Val Leu Ala
   65 70 75 80
   Ser Leu Arg Thr Val Arg Asn Asn Phe Ala Ala Leu Thr Asn Leu Gln
   85 90 95
   Asp Arg Ala Pro Ser Lys Arg Ser Pro Met Cys Asn Gln Pro Ser Ile
   100 105 110
   Asn Lys Ala Thr Ile Thr Glu Glu Ala Tyr Gln Lys Leu Ala Ser Glu
   115 120 125
   Thr Leu Glu Glu Leu Asp Trp Cys Leu Asp Gln Leu Glu Thr Leu Gln
   130 135 140
   Thr Arg His Ser Val Ser Glu Met Ala Ser Asn Lys Phe Lys Arg Met
   145 150 155 160
   Leu Asn Arg Glu Leu Thr His Leu Ser Glu Met Ser Arg Ser Gly Asn
   165 170 175
   Gln Val Ser Glu Phe Ile Ser Asn Thr Phe Leu Asp Lys Gln His Glu
   180 185 190
   Val Glu Ile Pro Ser Pro Thr Gln Lys Glu Lys Glu Lys Lys Lys Arg
   195 200 205
   Pro Met Ser Gln Ile Ser Gly Val Lys Lys Leu Met His Ser Ser Ser
   210 215 220
   Leu Thr Asn Ser Ser Ile Pro Arg Phe Gly Val Lys Thr Glu Gln Glu
   225 230 235 240
   Asp Val Leu Ala Lys Glu Leu Glu Asp Val Asn Lys Trp Gly Leu His
   245 250 255
   Val Phe Arg Ile Ala Glu Leu Ser Gly Asn Arg Pro Leu Thr Val Ile
   260 265 270
   Met His Thr Ile Phe Gln Glu Arg Asp Leu Leu Lys Thr Phe Lys Ile
   275 280 285
   Pro Val Asp Thr Leu Ile Thr Tyr Leu Met Thr Leu Glu Asp His Tyr
   290 295 300
   His Ala Asp Val Ala Tyr His Asn Asn Ile His Ala Ala Asp Val Val
   305 310 315 320
   Gln Ser Thr His Val Leu Leu Ser Thr Pro Ala Leu Glu Ala Val Phe
   325 330 335
   Thr Asp Leu Glu Ile Leu Ala Ala Ile Phe Ala Ser Ala Ile His Asp
   340 345 350
   Val Asp His Pro Gly Val Ser Asn Gln Phe Leu Ile Asn Thr Asn Ser
   355 360 365
   Glu Leu Ala Leu Met Tyr Asn Asp Ser Ser Val Leu Glu Asn His His
   370 375 380
   Leu Ala Val Gly Phe Lys Leu Leu Gln Glu Glu Asn Cys Asp Ile Phe
   385 390 395 400
   Gln Asn Leu Thr Lys Lys Gln Arg Gln Ser Leu Arg Lys Met Val Ile
   405 410 415
   Asp Ile Val Leu Ala Thr Asp Met Ser Lys His Met Asn Leu Leu Ala
   420 425 430
   Asp Leu Lys Thr Met Val Glu Thr Lys Lys Val Thr Ser Ser Gly Val
   435 440 445
   Leu Leu Leu Asp Asn Tyr Ser Asp Arg Ile Gln Val Leu Gln Asn Met
   450 455 460
   Val His Cys Ala Asp Leu Ser Asn Pro Thr Lys Pro Leu Gln Leu Tyr
   465 470 475 480
   Arg Gln Trp Thr Asp Arg Ile Met Glu Glu Phe Phe Arg Gln Gly Asp
   485 490 495
   Arg Glu Arg Glu Arg Gly Met Glu Ile Ser Pro Met Cys Asp Lys His
   500 505 510
   Asn Ala Ser Val Glu Lys Ser Gln Val Gly Phe Ile Asp Tyr Ile Val
   515 520 525
   His Pro Leu Trp Glu Thr Trp Ala Asp Leu Val His Pro Asp Ala Gln
   530 535 540
   Asp Ile Leu Asp Thr Leu Glu Asp Asn Arg Glu Trp Tyr Gln Ser Thr
   545 550 555 560
   Ile Pro Gln Ser Pro Ser Pro Ala Pro Asp Asp Pro Glu Glu Gly Arg
   565 570 575
   Gln Gly Gln Thr Glu Lys Phe Gln Phe Glu Leu Thr Leu Glu Glu Asp
   580 585 590
   Gly Glu Ser Asp Thr Glu Lys Asp Ser Gly Ser Gln Val Glu Glu Asp
   595 600 605
   Thr Ser Cys Ser Asp Ser Lys Thr Leu Cys Thr Gln Asp Ser Glu Ser
   610 615 620
   Thr Glu Ile Pro Leu Asp Glu Gln Val Glu Glu Glu Ala Val Gly Glu
   625 630 635 640
   Glu Glu Glu Ser Gln Pro Glu Ala Cys Val Ile Asp Asp Arg Ser Pro
   645 650 655
   Asp Thr His His His His His His
   660
<210> 3
<211> 747
<212> PRT
<213> Homo sapiens
<220>
<221> PDE4D5
<222> (1)..(747)
<223>
<220>
<221> isoform_specific_N-terminus
<222> (1)..(90)
<223>
<220>
<221> LF1_splice_site
<222> (91)..(91)
<223>
<220>
<221> UCR1_site
<222> (124)..(124)
<223>
<220>
<221> UCR1_site-13
<222> (111)..(111)
<223>
<220>
<221> Ser54
<222> (128)..(128)
<223>
<220>
<221> Ser579
<222> (653)..(653)
<223>
<400> 3
   Met Ala Gln Gln Thr Ser Pro Asp Thr Leu Thr Val Pro Glu Val Asp
   1 5 10 15
   Asn Pro His Cys Pro Asn Pro Trp Leu Asn Glu Asp Leu Val Lys Ser
   20 25 30
   Leu Arg Glu Asn Leu Leu Gln His Glu Lys Ser Lys Thr Ala Arg Lys
   35 40 45
   Ser Val Ser Pro Lys Leu Ser Pro Val Ile Ser Pro Arg Asn Ser Pro
   50 55 60
   Arg Leu Leu Arg Arg Met Leu Leu Ser Ser Asn Ile Pro Lys Gln Arg
   65 70 75 80
   Arg Phe Thr Val Ala His Thr Cys Lys Leu Phe Asp Val Asp Asn Gly
   85 90 95
   Thr Ser Ala Gly Arg Ser Pro Leu Asp Pro Met Thr Ser Pro Gly Ser
   100 105 110
   Gly Leu Ile Leu Gln Ala Asn Phe Val His Ser Gln Arg Arg Glu Ser
   115 120 125
   Phe Leu Tyr Arg Ser Asp Ser Asp Tyr Asp Leu Ser Pro Lys Ser Met
   130 135 140
   Ser Arg Asn Ser Ser Ile Ala Ser Asp Ile His Gly Asp Asp Leu Ile
   145 150 155 160
   Val Thr Pro Phe Ala Gln Val Leu Ala Ser Leu Arg Thr Val Arg Asn
   165 170 175
   Asn Phe Ala Ala Leu Thr Asn Leu Gln Asp Arg Ala Pro Ser Lys Arg
   180 185 190
   Ser Pro Met Cys Asn Gln Pro Ser Ile Asn Lys Ala Thr Ile Thr Glu
   195 200 205
   Glu Ala Tyr Gln Lys Leu Ala Ser Glu Thr Leu Glu Glu Leu Asp Trp
   210 215 220
   Cys Leu Asp Gln Leu Glu Thr Leu Gln Thr Arg His Ser Val Ser Glu
   225 230 235 240
   Met Ala Ser Asn Lys Phe Lys Arg Met Leu Asn Arg Glu Leu Thr His
   245 250 255
   Leu Ser Glu Met Ser Arg Ser Gly Asn Gln Val Ser Glu Phe Ile Ser
   260 265 270
   Asn Thr Phe Leu Asp Lys Gln His Glu Val Glu Ile Pro Ser Pro Thr
   275 280 285
   Gln Lys Glu Lys Glu Lys Lys Lys Arg Pro Met Ser Gln Ile Ser Gly
   290 295 300
   Val Lys Lys Leu Met His Ser Ser Ser Leu Thr Asn Ser Ser Ile Pro
   305 310 315 320
   Arg Phe Gly Val Lys Thr Glu Gln Glu Asp Val Leu Ala Lys Glu Leu
   325 330 335
   Glu Asp Val Asn Lys Trp Gly Leu His Val Phe Arg Ile Ala Glu Leu
   340 345 350
   Ser Gly Asn Arg Pro Leu Thr Val Ile Met His Thr Ile Phe Gln Glu
   355 360 365
   Arg Asp Leu Leu Lys Thr Phe Lys Ile Pro Val Asp Thr Leu Ile Thr
   370 375 380
   Tyr Leu Met Thr Leu Glu Asp His Tyr His Ala Asp Val Ala Tyr His
   385 390 395 400
   Asn Asn Ile His Ala Ala Asp Val Val Gln Ser Thr His Val Leu Leu
   405 410 415
   Ser Thr Pro Ala Leu Glu Ala Val Phe Thr Asp Leu Glu Ile Leu Ala
   420 425 430
   Ala Ile Phe Ala Ser Ala Ile His Asp Val Asp His Pro Gly Val Ser
   435 440 445
   Asn Gln Phe Leu Ile Asn Thr Asn Ser Glu Leu Ala Leu Met Tyr Asn
   450 455 460
   Asp Ser Ser Val Leu Glu Asn His His Leu Ala Val Gly Phe Lys Leu
   465 470 475 480
   Leu Gln Glu Glu Asn Cys Asp Ile Phe Gln Asn Leu Thr Lys Lys Gln
   485 490 495
   Arg Gln Ser Leu Arg Lys Met Val Ile Asp Ile Val Leu Ala Thr Asp
   500 505 510
   Met Ser Lys His Met Asn Leu Leu Ala Asp Leu Lys Thr Met Val Glu
   515 520 525
   Thr Lys Lys Val Thr Ser Ser Gly Val Leu Leu Leu Asp Asn Tyr Ser
   530 535 540
   Asp Arg Ile Gln Val Leu Gln Asn Met Val His Cys Ala Asp Leu Ser
   545 550 555 560
   Asn Pro Thr Lys Pro Leu Gln Leu Tyr Arg Gln Trp Thr Asp Arg Ile
   565 570 575
   Met Glu Glu Phe Phe Arg Gln Gly Asp Arg Glu Arg Glu Arg Gly Met
   580 585 590
   Glu Ile Ser Pro Met Cys Asp Lys His Asn Ala Ser Val Glu Lys Ser
   595 600 605
   Gln Val Gly Phe Ile Asp Tyr Ile Val His Pro Leu Trp Glu Thr Trp
   610 615 620
   Ala Asp Leu Val His Pro Asp Ala Gln Asp Ile Leu Asp Thr Leu Glu
   625 630 635 640
   Asp Asn Arg Glu Trp Tyr Gln Ser Thr Ile Pro Gln Ser Pro Ser Pro
   645 650 655
   Ala Pro Asp Asp Pro Glu Glu Gly Arg Gln Gly Gln Thr Glu Lys Phe
   660 665 670
   Gln Phe Glu Leu Thr Leu Glu Glu Asp Gly Glu Ser Asp Thr Glu Lys
   675 680 685
   Asp Ser Gly Ser Gln Val Glu Glu Asp Thr Ser Cys Ser Asp Ser Lys
   690 695 700
   Thr Leu Cys Thr Gln Asp Ser Glu Ser Thr Glu Ile Pro Leu Asp Glu
   705 710 715 720
   Gln Val Glu Glu Glu Ala Val Gly Glu Glu Glu Glu Ser Gln Pro Glu
   725 730 735
   Ala Cys Val Ile Asp Asp Arg Ser Pro Asp Thr
   740 745
<210> 4
<211> 689
<212> PRT
<213> Homo sapiens
<220>
<221> PDE4D6
<222> (1)..(689)
<223>
<220>
<221> isoform_specific_N-terminus
<222> (1)..(32)
<223>
<220>
<221> LF1_splice_site
<222> (33)..(33)
<223>
<220>
<221> UCR1_start
<222> (66)..(66)
<223>
<220>
<221> UCR1_start-13
<222> (53)..(53)
<223>
<220>
<221> Ser54
<222> (70)..(70)
<223>
<220>
<221> Ser579
<222> (595)..(595)
<223>
<400> 4
   Met Ala Phe Val Trp Asp Pro Leu Gly Ala Thr Val Pro Gly Pro Ser
   1 5 10 15
   Thr Arg Ala Lys Ser Arg Leu Arg Phe Ser Lys Ser Tyr Ser Lys Leu
   20 25 30
   Phe Asp Val Asp Asn Gly Thr Ser Ala Gly Arg Ser Pro Leu Asp Pro
   35 40 45
   Met Thr Ser Pro Gly Ser Gly Leu Ile Leu Gln Ala Asn Phe Val His
   50 55 60
   Ser Gln Arg Arg Glu Ser Phe Leu Tyr Arg Ser Asp Ser Asp Tyr Asp
   65 70 75 80
   Leu Ser Pro Lys Ser Met Ser Arg Asn Ser Ser Ile Ala Ser Asp Ile
   85 90 95
   His Gly Asp Asp Leu Ile Val Thr Pro Phe Ala Gln Val Leu Ala Ser
   100 105 110
   Leu Arg Thr Val Arg Asn Asn Phe Ala Ala Leu Thr Asn Leu Gln Asp
   115 120 125
   Arg Ala Pro Ser Lys Arg Ser Pro Met Cys Asn Gln Pro Ser Ile Asn
   130 135 140
   Lys Ala Thr Ile Thr Glu Glu Ala Tyr Gln Lys Leu Ala Ser Glu Thr
   145 150 155 160
   Leu Glu Glu Leu Asp Trp Cys Leu Asp Gln Leu Glu Thr Leu Gln Thr
   165 170 175
   Arg His Ser Val Ser Glu Met Ala Ser Asn Lys Phe Lys Arg Met Leu
   180 185 190
   Asn Arg Glu Leu Thr His Leu Ser Glu Met Ser Arg Ser Gly Asn Gln
   195 200 205
   Val Ser Glu Phe Ile Ser Asn Thr Phe Leu Asp Lys Gln His Glu Val
   210 215 220
   Glu Ile Pro Ser Pro Thr Gln Lys Glu Lys Glu Lys Lys Lys Arg Pro
   225 230 235 240
   Met Ser Gln Ile Ser Gly Val Lys Lys Leu Met His Ser Ser Ser Leu
   245 250 255
   Thr Asn Ser Ser Ile Pro Arg Phe Gly Val Lys Thr Glu Gln Glu Asp
   260 265 270
   Val Leu Ala Lys Glu Leu Glu Asp Val Asn Lys Trp Gly Leu His Val
   275 280 285
   Phe Arg Ile Ala Glu Leu Ser Gly Asn Arg Pro Leu Thr Val Ile Met
   290 295 300
   His Thr Ile Phe Gln Glu Arg Asp Leu Leu Lys Thr Phe Lys Ile Pro
   305 310 315 320
   Val Asp Thr Leu Ile Thr Tyr Leu Met Thr Leu Glu Asp His Tyr His
   325 330 335
   Ala Asp Val Ala Tyr His Asn Asn Ile His Ala Ala Asp Val Val Gln
   340 345 350
   Ser Thr His Val Leu Leu Ser Thr Pro Ala Leu Glu Ala Val Phe Thr
   355 360 365
   Asp Leu Glu Ile Leu Ala Ala Ile Phe Ala Ser Ala Ile His Asp Val
   370 375 380
   Asp His Pro Gly Val Ser Asn Gln Phe Leu Ile Asn Thr Asn Ser Glu
   385 390 395 400
   Leu Ala Leu Met Tyr Asn Asp Ser Ser Val Leu Glu Asn His His Leu
   405 410 415
   Ala Val Gly Phe Lys Leu Leu Gln Glu Glu Asn Cys Asp Ile Phe Gln
   420 425 430
   Asn Leu Thr Lys Lys Gln Arg Gln Ser Leu Arg Lys Met Val Ile Asp
   435 440 445
   Ile Val Leu Ala Thr Asp Met Ser Lys His Met Asn Leu Leu Ala Asp
   450 455 460
   Leu Lys Thr Met Val Glu Thr Lys Lys Val Thr Ser Ser Gly Val Leu
   465 470 475 480
   Leu Leu Asp Asn Tyr Ser Asp Arg Ile Gln Val Leu Gln Asn Met Val
   485 490 495
   His Cys Ala Asp Leu Ser Asn Pro Thr Lys Pro Leu Gln Leu Tyr Arg
   500 505 510
   Gln Trp Thr Asp Arg Ile Met Glu Glu Phe Phe Arg Gln Gly Asp Arg
   515 520 525
   Glu Arg Glu Arg Gly Met Glu Ile Ser Pro Met Cys Asp Lys His Asn
   530 535 540
   Ala Ser Val Glu Lys Ser Gln Val Gly Phe Ile Asp Tyr Ile Val His
   545 550 555 560
   Pro Leu Trp Glu Thr Trp Ala Asp Leu Val His Pro Asp Ala Gln Asp
   565 570 575
   Ile Leu Asp Thr Leu Glu Asp Asn Arg Glu Trp Tyr Gln Ser Thr Ile
   580 585 590
   Pro Gln Ser Pro Ser Pro Ala Pro Asp Asp Pro Glu Glu Gly Arg Gln
   595 600 605
   Gly Gln Thr Glu Lys Phe Gln Phe Glu Leu Thr Leu Glu Glu Asp Gly
   610 615 620
   Glu Ser Asp Thr Glu Lys Asp Ser Gly Ser Gln Val Glu Glu Asp Thr
   625 630 635 640
   Ser Cys Ser Asp Ser Lys Thr Leu Cys Thr Gln Asp Ser Glu Ser Thr
   645 650 655
   Glu Ile Pro Leu Asp Glu Gln Val Glu Glu Glu Ala Val Gly Glu Glu
   660 665 670
   Glu Glu Ser Gln Pro Glu Ala Cys Val Ile Asp Asp Arg Ser Pro Asp
   675 680 685
   Thr
<210> 5
<211> 750
<212> PRT
<213> Homo sapiens
<220>
<221> PDE4D7
<222> (1)..(750)
<223>
<220>
<221> isoform_specific_N-terminus
<222> (1)..(93)
<223>
<220>
<221> LF1_splice_site
<222> (94)..(94)
<223>
<220>
<221> UCR1_start
<222> (127)..(127)
<223>
<220>
<221> UCR1_start-13
<222> (114)..(114)
<223>
<220>
<221> Ser54
<222> (131)..(131)
<223>
<220>
<221> Ser579
<222> (656)..(656)
<223>
<400> 5
   Met Lys Arg Asn Thr Cys Asp Leu Leu Ser Arg Ser Lys Ser Ala Ser
   1               5                   10                  15
   Glu Glu Thr Leu His Ser Ser Asn Glu Glu Glu Asp Pro Phe Arg Gly
               20                  25                  30
   Met Glu Pro Tyr Leu Val Arg Arg Leu Ser Cys Arg Asn Ile Gln Leu
           35                  40                  45
   Pro Pro Leu Ala Phe Arg Gln Leu Glu Gln Ala Asp Leu Lys Ser Glu
   50 55 60
   Ser Glu Asn Ile Gln Arg Pro Thr Ser Leu Pro Leu Lys Ile Leu Pro
   65 70 75 80
   Leu Ile Ala Ile Thr Ser Ala Glu Ser Ser Gly Lys Leu Phe Asp Val
   85 90 95
   Asp Asn Gly Thr Ser Ala Gly Arg Ser Pro Leu Asp Pro Met Thr Ser
   100 105 110
   Pro Gly Ser Gly Leu Ile Leu Gln Ala Asn Phe Val His Ser Gln Arg
   115 120 125
   Arg Glu Ser Phe Leu Tyr Arg Ser Asp Ser Asp Tyr Asp Leu Ser Pro
   130 135 140
   Lys Ser Met Ser Arg Asn Ser Ser Ile Ala Ser Asp Ile His Gly Asp
   145 150 155 160
   Asp Leu Ile Val Thr Pro Phe Ala Gln Val Leu Ala Ser Leu Arg Thr
   165 170 175
   Val Arg Asn Asn Phe Ala Ala Leu Thr Asn Leu Gln Asp Arg Ala Pro
   180 185 190
   Ser Lys Arg Ser Pro Met Cys Asn Gln Pro Ser Ile Asn Lys Ala Thr
   195 200 205
   Ile Thr Glu Glu Ala Tyr Gln Lys Leu Ala Ser Glu Thr Leu Glu Glu
   210 215 220
   Leu Asp Trp Cys Leu Asp Gln Leu Glu Thr Leu Gln Thr Arg His Ser
   225 230 235 240
   Val Ser Glu Met Ala Ser Asn Lys Phe Lys Arg Met Leu Asn Arg Glu
   245 250 255
   Leu Thr His Leu Ser Glu Met Ser Arg Ser Gly Asn Gln Val Ser Glu
   260 265 270
   Phe Ile Ser Asn Thr Phe Leu Asp Lys Gln His Glu Val Glu Ile Pro
   275 280 285
   Ser Pro Thr Gln Lys Glu Lys Glu Lys Lys Lys Arg Pro Met Ser Gln
   290 295 300
   Ile Ser Gly Val Lys Lys Leu Met His Ser Ser Ser Leu Thr Asn Ser
   305 310 315 320
   Ser Ile Pro Arg Phe Gly Val Lys Thr Glu Gln Glu Asp Val Leu Ala
   325 330 335
   Lys Glu Leu Glu Asp Val Asn Lys Trp Gly Leu His Val Phe Arg Ile
   340 345 350
   Ala Glu Leu Ser Gly Asn Arg Pro Leu Thr Val Ile Met His Thr Ile
   355 360 365
   Phe Gln Glu Arg Asp Leu Leu Lys Thr Phe Lys Ile Pro Val Asp Thr
   370 375 380
   Leu Ile Thr Tyr Leu Met Thr Leu Glu Asp His Tyr His Ala Asp Val
   385 390 395 400
   Ala Tyr His Asn Asn Ile His Ala Ala Asp Val Val Gln Ser Thr His
   405 410 415
   Val Leu Leu Ser Thr Pro Ala Leu Glu Ala Val Phe Thr Asp Leu Glu
   420 425 430
   Ile Leu Ala Ala Ile Phe Ala Ser Ala Ile His Asp Val Asp His Pro
   435 440 445
   Gly Val Ser Asn Gln Phe Leu Ile Asn Thr Asn Ser Glu Leu Ala Leu
   450 455 460
   Met Tyr Asn Asp Ser Ser Val Leu Glu Asn His His Leu Ala Val Gly
   465 470 475 480
   Phe Lys Leu Leu Gln Glu Glu Asn Cys Asp Ile Phe Gln Asn Leu Thr
   485 490 495
   Lys Lys Gln Arg Gln Ser Leu Arg Lys Met Val Ile Asp Ile Val Leu
   500 505 510
   Ala Thr Asp Met Ser Lys His Met Asn Leu Leu Ala Asp Leu Lys Thr
   515 520 525
   Met Val Glu Thr Lys Lys Val Thr Ser Ser Gly Val Leu Leu Leu Asp
   530 535 540
   Asn Tyr Ser Asp Arg Ile Gln Val Leu Gln Asn Met Val His Cys Ala
   545 550 555 560
   Asp Leu Ser Asn Pro Thr Lys Pro Leu Gln Leu Tyr Arg Gln Trp Thr
   565 570 575
   Asp Arg Ile Met Glu Glu Phe Phe Arg Gln Gly Asp Arg Glu Arg Glu
   580 585 590
   Arg Gly Met Glu Ile Ser Pro Met Cys Asp Lys His Asn Ala Ser Val
   595 600 605
   Glu Lys Ser Gln Val Gly Phe Ile Asp Tyr Ile Val His Pro Leu Trp
   610 615 620
   Glu Thr Trp Ala Asp Leu Val His Pro Asp Ala Gln Asp Ile Leu Asp
   625 630 635 640
   Thr Leu Glu Asp Asn Arg Glu Trp Tyr Gln Ser Thr Ile Pro Gln Ser
   645 650 655
   Pro Ser Pro Ala Pro Asp Asp Pro Glu Glu Gly Arg Gln Gly Gln Thr
   660 665 670
   Glu Lys Phe Gln Phe Glu Leu Thr Leu Glu Glu Asp Gly Glu Ser Asp
   675 680 685
   Thr Glu Lys Asp Ser Gly Ser Gln Val Glu Glu Asp Thr Ser Cys Ser
   690 695 700
   Asp Ser Lys Thr Leu Cys Thr Gln Asp Ser Glu Ser Thr Glu Ile Pro
   705 710 715 720
   Leu Asp Glu Gln Val Glu Glu Glu Ala Val Gly Glu Glu Glu Glu Ser
   725 730 735
   Gln Pro Glu Ala Cys Val Ile Asp Asp Arg Ser Pro Asp Thr
   740 745 750
<210> 6
<211> 679
<212> PRT
<213> Homo sapiens
<220>
<221> PDE4D8
<222> (1)..(679)
<223>
<220>
<221> isoform_specific_N-terminus
<222> (1)..(23)
<223>
<220>
<221> LF1_splice_site
<222> (23)..(23)
<223>
<220>
<221> UCR1_start
<222> (56)..(56)
<223>
<220>
<221> UCR1_start-13
<222> (43)..(43)
<223>
<220>
<221> Ser54
<222> (60)..(60)
<223>
<220>
<221> Ser579
<222> (585)..(585)
<223>
<400> 6
   Met Ser Ile Ile Met Lys Pro Arg Ser Arg Ser Thr Ser Ser Leu Arg
   1 5 10 15
   Thr Ala Glu Ala Lys Leu Phe Asp Val Asp Asn Gly Thr Ser Ala Gly
   20 25 30
   Arg Ser Pro Leu Asp Pro Met Thr Ser Pro Gly Ser Gly Leu Ile Leu
   35 40 45
   Gln Ala Asn Phe Val His Ser Gln Arg Arg Glu Ser Phe Leu Tyr Arg
   50 55 60
   Ser Asp Ser Asp Tyr Asp Leu Ser Pro Lys Ser Met Ser Arg Asn Ser
   65 70 75 80
   Ser Ile Ala Ser Asp Ile His Gly Asp Asp Leu Ile Val Thr Pro Phe
   85 90 95
   Ala Gln Val Leu Ala Ser Leu Arg Thr Val Arg Asn Asn Phe Ala Ala
   100 105 110
   Leu Thr Asn Leu Gln Asp Arg Ala Pro Ser Lys Arg Ser Pro Met Cys
   115 120 125
   Asn Gln Pro Ser Ile Asn Lys Ala Thr Ile Thr Glu Glu Ala Tyr Gln
   130 135 140
   Lys Leu Ala Ser Glu Thr Leu Glu Glu Leu Asp Trp Cys Leu Asp Gln
   145 150 155 160
   Leu Glu Thr Leu Gln Thr Arg His Ser Val Ser Glu Met Ala Ser Asn
   165 170 175
   Lys Phe Lys Arg Met Leu Asn Arg Glu Leu Thr His Leu Ser Glu Met
   180 185 190
   Ser Arg Ser Gly Asn Gln Val Ser Glu Phe Ile Ser Asn Thr Phe Leu
   195 200 205
   Asp Lys Gln His Glu Val Glu Ile Pro Ser Pro Thr Gln Lys Glu Lys
   210 215 220
   Glu Lys Lys Lys Arg Pro Met Ser Gln Ile Ser Gly Val Lys Lys Leu
   225 230 235 240
   Met His Ser Ser Ser Leu Thr Asn Ser Ser Ile Pro Arg Phe Gly Val
   245 250 255
   Lys Thr Glu Gln Glu Asp Val Leu Ala Lys Glu Leu Glu Asp Val Asn
   260 265 270
   Lys Trp Gly Leu His Val Phe Arg Ile Ala Glu Leu Ser Gly Asn Arg
   275 280 285
   Pro Leu Thr Val Ile Met His Thr Ile Phe Gln Glu Arg Asp Leu Leu
   290 295 300
   Lys Thr Phe Lys Ile Pro Val Asp Thr Leu Ile Thr Tyr Leu Met Thr
   305 310 315 320
   Leu Glu Asp His Tyr His Ala Asp Val Ala Tyr His Asn Asn Ile His
   325 330 335
   Ala Ala Asp Val Val Gln Ser Thr His Val Leu Leu Ser Thr Pro Ala
   340 345 350
   Leu Glu Ala Val Phe Thr Asp Leu Glu Ile Leu Ala Ala Ile Phe Ala
   355 360 365
   Ser Ala Ile His Asp Val Asp His Pro Gly Val Ser Asn Gln Phe Leu
   370 375 380
   Ile Asn Thr Asn Ser Glu Leu Ala Leu Met Tyr Asn Asp Ser Ser Val
   385 390 395 400
   Leu Glu Asn His His Leu Ala Val Gly Phe Lys Leu Leu Gln Glu Glu
   405 410 415
   Asn Cys Asp Ile Phe Gln Asn Leu Thr Lys Lys Gln Arg Gln Ser Leu
   420 425 430
   Arg Lys Met Val Ile Asp Ile Val Leu Ala Thr Asp Met Ser Lys His
   435 440 445
   Met Asn Leu Leu Ala Asp Leu Lys Thr Met Val Glu Thr Lys Lys Val
   450 455 460
   Thr Ser Ser Gly Val Leu Leu Leu Asp Asn Tyr Ser Asp Arg Ile Gln
   465 470 475 480
   Val Leu Gln Asn Met Val His Cys Ala Asp Leu Ser Asn Pro Thr Lys
   485 490 495
   Pro Leu Gln Leu Tyr Arg Gln Trp Thr Asp Arg Ile Met Glu Glu Phe
   500 505 510
   Phe Arg Gln Gly Asp Arg Glu Arg Glu Arg Gly Met Glu Ile Ser Pro
   515 520 525
   Met Cys Asp Lys His Asn Ala Ser Val Glu Lys Ser Gln Val Gly Phe
   530 535 540
   Ile Asp Tyr Ile Val His Pro Leu Trp Glu Thr Trp Ala Asp Leu Val
   545 550 555 560
   His Pro Asp Ala Gln Asp Ile Leu Asp Thr Leu Glu Asp Asn Arg Glu
   565 570 575
   Trp Tyr Gln Ser Thr Ile Pro Gln Ser Pro Ser Pro Ala Pro Asp Asp
   580 585 590
   Pro Glu Glu Gly Arg Gln Gly Gln Thr Glu Lys Phe Gln Phe Glu Leu
   595 600 605
   Thr Leu Glu Glu Asp Gly Glu Ser Asp Thr Glu Lys Asp Ser Gly Ser
   610 615 620
   Gln Val Glu Glu Asp Thr Ser Cys Ser Asp Ser Lys Thr Leu Cys Thr
   625 630 635 640
   Gln Asp Ser Glu Ser Thr Glu Ile Pro Leu Asp Glu Gln Val Glu Glu
   645 650 655
   Glu Ala Val Gly Glu Glu Glu Glu Ser Gln Pro Glu Ala Cys Val Ile
   660 665 670
   Asp Asp Arg Ser Pro Asp Thr
   675
<210> 7
<211> 809
<212> PRT
<213> Homo sapiens
<220>
<221> PDE4D4
<222> (1)..(809)
<223>
<220>
<221> LF1_splice_site
<222> (153)..(153)
<223>
<220>
<221> UCR_1_start
<222> (186)..(186)
<223>
<220>
<221> UCR1_start_-13
<222> (173)..(173)
<223>
<220>
<221> Ser54
<222> (190)..(190)
<223>
<220>
<221> Ser579
<222> (715)..(715)
<223>
<400> 7
<210> 8
<211> 1974
<212> DNA
<213> Homo sapiens
<220>
<221> PDE4D_core_contruct
<222> (1)..(1974)
<223>
<400> 8
   tttgatgtgg acaatggcac atctgcggga cggagtccct tggatcccat gaccagccca     60
   ggatccgggc taattctcca agcaaatttt gtccacagtc aacgacggga gtccttcctg    120
   tatcgatccg acagcgatta tgacctctct ccaaagtcta tgtcccggaa ctcctccatt    180
   gccagtgata tacacggaga tgacttgatt gtgactccat ttgctcaggt cttggccagt    240
   ctgcgaactg tacgaaacaa ctttgctgca ttaactaatt tgcaagatcg agcacctagc    300
   aaaagatcac ccatgtgcaa ccaaccatcc atcaacaaag ccaccataac agaggaggcc    360
   taccagaaac tggccagcga gaccctggag gagctggact ggtgtctgga ccagctagag    420
   accctacaga ccaggcactc cgtcagtgag atggcctcca acaagtttaa aaggatgctt    480
   aatcgggagc tcacccatct ctctgaaatg agtcggtctg gaaatcaagt gtcagagttt    540
   atatcaaaca cattcttaga taagcaacat gaagtggaaa ttccttctcc aactcagaag    600
   gaaaaggaga aaaagaaaag accaatgtct cagatcagtg gagtcaagaa attgatgcac      660
   agctctagtc tgactaattc aagtatccca aggtttggag ttaaaactga acaagaagat      720
   gtccttgcca aggaactaga agatgtgaac aaatggggtc ttcatgtttt cagaatagca      780
   gagttgtctg gtaaccggcc cttgactgtt atcatgcaca ccatttttca ggaacgggat      840
   ttattaaaaa catttaaaat tccagtagat actttaatta catatcttat gactctcgaa      900
   gaccattacc atgctgatgt ggcctatcac aacaatatcc atgctgcaga tgttgtccag      960
   tctactcatg tgctattatc tacacctgct ttggaggctg tgtttacaga tttggagatt     1020
   cttgcagcaa tttttgccag tgcaatacat gatgtagatc atcctggtgt gtccaatcaa     1080
   tttctgatca atacaaactc tgaacttgcc ttgatgtaca atgattcctc agtcttagag     1140
   aaccatcatt tggctgtggg ctttaaattg cttcaggaag aaaactgtga cattttccag     1200
   aatttgacca aaaaacaaag acaatcttta aggaaaatgg tcattgacat cgtacttgca     1260
   acagatatgt caaaacacat gaatctactg gctgatttga agactatggt tgaaactaag     1320
   aaagtgacaa gctctggagt tcttcttctt gataattatt ccgataggat tcaggttctt     1380
   cagaatatgg tgcactgtgc agatctgagc aacccaacaa agcctctcca gctgtaccgc     1440
   cagtggacgg accggataat ggaggagttc ttccgccaag gagaccgaga gagggaacgt     1500
   ggcatggaga taagccccat gtgtgacaag cacaatgctt ccgtggaaaa atcacaggtg     1560
   ggcttcatag actatattgt tcatcccctc tgggagacat gggcagacct cgtccaccct     1620
   gacgcccagg atattttgga cactttggag gacaatcgtg aatggtacca gagcacaatc     1680
   cctcagagcc cctctcctgc acctgatgac ccagaggagg gccggcaggg tcaaactgag     1740
   aaattccagt ttgaactaac tttagaggaa gatggtgagt cagacacgga aaaggacagt     1800
   ggcagtcaag tggaagaaga cactagctgc agtgactcca agactctttg tactcaagac     1860
   tcagagtcta ctgaaattcc ccttgatgaa caggttgaag aggaggcagt aggggaagaa     1920
   gaggaaagcc agcctgaagc ctgtgtcata gatgatcgtt ctcctgacac gtaa           1974

### SEQUENCE LISTING

<110> F. Hoffmann-La Roche AG
<120> Modified PDE4D polypeptides
<130> case 21507
<160> 6
<170> PatentIn version 3.1
<210> 1
<211> 673
<212> PRT
<213> Homo sapiens
<220>
<221> PDE4D3
<222> (1)..(673)
<223>
<220>
<221> LF1_splice_site
<222> (17)..(17)
<223>
<220>
<221> Ser579
<222> (579)..(579)
<223>
<220>
<221> Ser54
<222> (54)..(54)
<223>
<220>
<221> UCR1_site_-13
<222> (37)..(37)
<223>
<220>
<221> UCR1_start
<222> (50)..(50)
<223>
<400> 1
   Met Met His Val Asn Asn Phe Pro Phe Arg Arg His Ser Trp Ile Cys
   1 5 10 15
   Phe Asp Val Asp Asn Gly Thr Ser Ala Gly Arg Ser Pro Leu Asp Pro
   20 25 30
   Met Thr Ser Pro Gly Ser Gly Leu Ile Leu Gln Ala Asn Phe Val His
   35 40 45
   Ser Gln Arg Arg Glu Ser Phe Leu Tyr Arg Ser Asp Ser Asp Tyr Asp
   50 55 60
   Leu Ser Pro Lys Ser Met Ser Arg Asn Ser Ser Ile Ala Ser Asp Ile
   65 70 75 80
   His Gly Asp Asp Leu Ile Val Thr Pro Phe Ala Gln Val Leu Ala Ser
   85 90 95
   Leu Arg Thr Val Arg Asn Asn Phe Ala Ala Leu Thr Asn Leu Gln Asp
   100 105 110
   Arg Ala Pro Ser Lys Arg Ser Pro Met Cys Asn Gln Pro Ser Ile Asn
   115 120 125
   Lys Ala Thr Ile Thr Glu Glu Ala Tyr Gln Lys Leu Ala Ser Glu Thr
   130 135 140
   Leu Glu Glu Leu Asp Trp Cys Leu Asp Gln Leu Glu Thr Leu Gln Thr
   145 150 155 160
   Arg His Ser Val Ser Glu Met Ala Ser Asn Lys Phe Lys Arg Met Leu
   165 170 175
   Asn Arg Glu Leu Thr His Leu Ser Glu Met Ser Arg Ser Gly Asn Gln
   180 185 190
   Val Ser Glu Phe Ile Ser Asn Thr Phe Leu Asp Lys Gln His Glu Val
   195 200 205
   Glu Ile Pro Ser Pro Thr Gln Lys Glu Lys Glu Lys Lys Lys Arg Pro
   210 215 220
   Met Ser Gln Ile Ser Gly Val Lys Lys Leu Met His Ser Ser Ser Leu
   225 230 235 240
   Thr Asn Ser Ser Ile Pro Arg Phe Gly Val Lys Thr Glu Gln Glu Asp
   245 250 255
   Val Leu Ala Lys Glu Leu Glu Asp Val Asn Lys Trp Gly Leu His Val
   260 265 270
   Phe Arg Ile Ala Glu Leu Ser Gly Asn Arg Pro Leu Thr Val Ile Met
   275 280 285
   His Thr Ile Phe Gln Glu Arg Asp Leu Leu Lys Thr Phe Lys Ile Pro
   290 295 300
   Val Asp Thr Leu Ile Thr Tyr Leu Met Thr Leu Glu Asp His Tyr His
   305 310 315 320
   Ala Asp Val Ala Tyr His Asn Asn Ile His Ala Ala Asp Val Val Gln
   325 330 335
   Ser Thr His Val Leu Leu Ser Thr Pro Ala Leu Glu Ala Val Phe Thr
   340 345 350
   Asp Leu Glu Ile Leu Ala Ala Ile Phe Ala Ser Ala Ile His Asp Val
   355 360 365
   Asp His Pro Gly Val Ser Asn Gln Phe Leu Ile Asn Thr Asn Ser Glu
   370 375 380
   Leu Ala Leu Met Tyr Asn Asp Ser Ser Val Leu Glu Asn His His Leu
   385 390 395 400
   Ala Val Gly Phe Lys Leu Leu Gln Glu Glu Asn Cys Asp Ile Phe Gln
   405 410 415
   Asn Leu Thr Lys Lys Gln Arg Gln Ser Leu Arg Lys Met Val Ile Asp
   420 425 430
   Ile Val Leu Ala Thr Asp Met Ser Lys His Met Asn Leu Leu Ala Asp
   435 440 445
   Leu Lys Thr Met Val Glu Thr Lys Lys Val Thr Ser Ser Gly Val Leu
   450 455 460
   Leu Leu Asp Asn Tyr Ser Asp Arg Ile Gln Val Leu Gln Asn Met Val
   465 470 475 480
   His Cys Ala Asp Leu Ser Asn Pro Thr Lys Pro Leu Gln Leu Tyr Arg
   485 490 495
   Gln Trp Thr Asp Arg Ile Met Glu Glu Phe Phe Arg Gln Gly Asp Arg
   500 505 510
   Glu Arg Glu Arg Gly Met Glu Ile Ser Pro Met Cys Asp Lys His Asn
   515 520 525
   Ala Ser Val Glu Lys Ser Gln Val Gly Phe Ile Asp Tyr Ile Val His
   530 535 540
   Pro Leu Trp Glu Thr Trp Ala Asp Leu Val His Pro Asp Ala Gln Asp
   545 550 555 560
   Ile Leu Asp Thr Leu Glu Asp Asn Arg Glu Trp Tyr Gln Ser Thr Ile
   565 570 575
   Pro Gln Ser Pro Ser Pro Ala Pro Asp Asp Pro Glu Glu Gly Arg Gln
   580 585 590
   Gly Gln Thr Glu Lys Phe Gln Phe Glu Leu Thr Leu Glu Glu Asp Gly
   595 600 605
   Glu Ser Asp Thr Glu Lys Asp Ser Gly Ser Gln Val Glu Glu Asp Thr
   610 615 620
   Ser Cys Ser Asp Ser Lys Thr Leu Cys Thr Gln Asp Ser Glu Ser Thr
   625 630 635 640
   Glu Ile Pro Leu Asp Glu Gln Val Glu Glu Glu Ala Val Gly Glu Glu
   645 650 655
   Glu Glu Ser Gln Pro Glu Ala Cys Val Ile Asp Asp Arg Ser Pro Asp
   660 665 670
   Thr
<210> 2
<211> 664
<212> PRT
<213> Homo sapiens
<220>
<221> PDE4D_core
<222> (1)..(664)
<223>
<220>
<221> LF1_splice_site
<222> (2)..(2)
<223>
<220>
<221> UCR1_site
<222> (35)..(35)
<223>
<220>
<221> UCR1_site_-13
<222> (22)..(22)
<223>
<220>
<221> SER54
<222> (39)..(39)
<223>
<220>
<221> SER579
<222> (564)..(564)
<223>
<400> 2
   Met Phe Asp Val Asp Asn Gly Thr Ser Ala Gly Arg Ser Pro Leu Asp
   1 5 10 15
   Pro Met Thr Ser Pro Gly Ser Gly Leu Ile Leu Gln Ala Asn Phe Val
   20 25 30
   His Ser Gln Arg Arg Glu Ser Phe Leu Tyr Arg Ser Asp Ser Asp Tyr
   35 40 45
   Asp Leu Ser Pro Lys Ser Met Ser Arg Asn Ser Ser Ile Ala Ser Asp
   50 55 60
   Ile His Gly Asp Asp Leu Ile Val Thr Pro Phe Ala Gln Val Leu Ala
   65 70 75 80
   Ser Leu Arg Thr Val Arg Asn Asn Phe Ala Ala Leu Thr Asn Leu Gln
   85 90 95
   Asp Arg Ala Pro Ser Lys Arg Ser Pro Met Cys Asn Gln Pro Ser Ile
   100 105 110
   Asn Lys Ala Thr Ile Thr Glu Glu Ala Tyr Gln Lys Leu Ala Ser Glu
   115 120 125
   Thr Leu Glu Glu Leu Asp Trp Cys Leu Asp Gln Leu Glu Thr Leu Gln
   130 135 140
   Thr Arg His Ser Val Ser Glu Met Ala Ser Asn Lys Phe Lys Arg Met
   145 150 155 160
   Leu Asn Arg Glu Leu Thr His Leu Ser Glu Met Ser Arg Ser Gly Asn
   165 170 175
   Gln Val Ser Glu Phe Ile Ser Asn Thr Phe Leu Asp Lys Gln His Glu
   180 185 190
   Val Glu Ile Pro Ser Pro Thr Gln Lys Glu Lys Glu Lys Lys Lys Arg
   195 200 205
   Pro Met Ser Gln Ile Ser Gly Val Lys Lys Leu Met His Ser Ser Ser
   210 215 220
   Leu Thr Asn Ser Ser Ile Pro Arg Phe Gly Val Lys Thr Glu Gln Glu
   225 230 235 240
   Asp Val Leu Ala Lys Glu Leu Glu Asp Val Asn Lys Trp Gly Leu His
   245 250 255
   Val Phe Arg Ile Ala Glu Leu Ser Gly Asn Arg Pro Leu Thr Val Ile
   260 265 270
   Met His Thr Ile Phe Gln Glu Arg Asp Leu Leu Lys Thr Phe Lys Ile
   275 280 285
   Pro Val Asp Thr Leu Ile Thr Tyr Leu Met Thr Leu Glu Asp His Tyr
   290 295 300
   His Ala Asp Val Ala Tyr His Asn Asn Ile His Ala Ala Asp Val Val
   305 310 315 320
   Gln Ser Thr His Val Leu Leu Ser Thr Pro Ala Leu Glu Ala Val Phe
   325 330 335
   Thr Asp Leu Glu Ile Leu Ala Ala Ile Phe Ala Ser Ala Ile His Asp
   340 345 350
   Val Asp His Pro Gly Val Ser Asn Gln Phe Leu Ile Asn Thr Asn Ser
   355 360 365
   Glu Leu Ala Leu Met Tyr Asn Asp Ser Ser Val Leu Glu Asn His His
   370 375 380
   Leu Ala Val Gly Phe Lys Leu Leu Gln Glu Glu Asn Cys Asp Ile Phe
   385 390 395 400
   Gln Asn Leu Thr Lys Lys Gln Arg Gln Ser Leu Arg Lys Met Val Ile
   405 410 415
   Asp Ile Val Leu Ala Thr Asp Met Ser Lys His Met Asn Leu Leu Ala
   420 425 430
   Asp Leu Lys Thr Met Val Glu Thr Lys Lys Val Thr Ser Ser Gly Val
   435 440 445
   Leu Leu Leu Asp Asn Tyr Ser Asp Arg Ile Gln Val Leu Gln Asn Met
   450 455 460
   Val His Cys Ala Asp Leu Ser Asn Pro Thr Lys Pro Leu Gln Leu Tyr
   465 470 475 480
   Arg Gln Trp Thr Asp Arg Ile Met Glu Glu Phe Phe Arg Gln Gly Asp
   485 490 495
   Arg Glu Arg Glu Arg Gly Met Glu Ile Ser Pro Met Cys Asp Lys His
   500 505 510
   Asn Ala Ser Val Glu Lys Ser Gln Val Gly Phe Ile Asp Tyr Ile Val
   515 520 525
   His Pro Leu Trp Glu Thr Trp Ala Asp Leu Val His Pro Asp Ala Gln
   530 535 540
   Asp Ile Leu Asp Thr Leu Glu Asp Asn Arg Glu Trp Tyr Gln Ser Thr
   545 550 555 560
   Ile Pro Gln Ser Pro Ser Pro Ala Pro Asp Asp Pro Glu Glu Gly Arg
   565 570 575
   Gln Gly Gln Thr Glu Lys Phe Gln Phe Glu Leu Thr Leu Glu Glu Asp
   580 585 590
   Gly Glu Ser Asp Thr Glu Lys Asp Ser Gly Ser Gln Val Glu Glu Asp
   595 600 605
   Thr Ser Cys Ser Asp Ser Lys Thr Leu Cys Thr Gln Asp Ser Glu Ser
   610 615 620
   Thr Glu Ile Pro Leu Asp Glu Gln Val Glu Glu Glu Ala Val Gly Glu
   625 630 635 640
   Glu Glu Glu Ser Gln Pro Glu Ala Cys Val Ile Asp Asp Arg Ser Pro
   645 650 655
   Asp Thr His His His His His His
   660
<210> 3
<211> 747
<212> PRT
<213> Homo sapiens
<220>
<221> PDE4D5
<222> (1)..(747)
<223>
<220>
<221> isoform_specific_N-terminus
<222> (1)..(90)
<223>
<220>
<221> LF1_splice_site
<222> (91)..(91)
<223>
<220>
<221> UCR1_site
<222> (124)..(124)
<223>
<220>
<221> UCR1_site-13
<222> (111)..(111)
<223>
<220>
<221> Ser54
<222> (128)..(128)
<223>
<220>
<221> Ser579
<222> (653)..(653)
<223>
<400> 3
   Met Ala Gln Gln Thr Ser Pro Asp Thr Leu Thr Val Pro Glu Val Asp
   1               5                   10                  15
   Asn Pro His Cys Pro Asn Pro Trp Leu Asn Glu Asp Leu Val Lys Ser
               20                  25                  30
   Leu Arg Glu Asn Leu Leu Gln His Glu Lys Ser Lys Thr Ala Arg Lys
           35                  40                  45
   Ser Val Ser Pro Lys Leu Ser Pro Val Ile Ser Pro Arg Asn Ser Pro
       50                  55                  60
   Arg Leu Leu Arg Arg Met Leu Leu Ser Ser Asn Ile Pro Lys Gln Arg
   65                  70                  75                  80
   Arg Phe Thr Val Ala His Thr Cys Lys Leu Phe Asp Val Asp Asn Gly
                   85                  90                  95
   Thr Ser Ala Gly Arg Ser Pro Leu Asp Pro Met Thr Ser Pro Gly Ser
               100                 105                 110
   Gly Leu Ile Leu Gln Ala Asn Phe Val His Ser Gln Arg Arg Glu Ser
           115                 120                 125
   Phe Leu Tyr Arg Ser Asp Ser Asp Tyr Asp Leu Ser Pro Lys Ser Met
       130                 135                 140
   Ser Arg Asn Ser Ser Ile Ala Ser Asp Ile His Gly Asp Asp Leu Ile
   145 150 155 160
   Val Thr Pro Phe Ala Gln Val Leu Ala Ser Leu Arg Thr Val Arg Asn
   165 170 175
   Asn Phe Ala Ala Leu Thr Asn Leu Gln Asp Arg Ala Pro Ser Lys Arg
   180 185 190
   Ser Pro Met Cys Asn Gln Pro Ser Ile Asn Lys Ala Thr Ile Thr Glu
   195 200 205
   Glu Ala Tyr Gln Lys Leu Ala Ser Glu Thr Leu Glu Glu Leu Asp Trp
   210 215 220
   Cys Leu Asp Gln Leu Glu Thr Leu Gln Thr Arg His Ser Val Ser Glu
   225 230 235 240
   Met Ala Ser Asn Lys Phe Lys Arg Met Leu Asn Arg Glu Leu Thr His
   245 250 255
   Leu Ser Glu Met Ser Arg Ser Gly Asn Gln Val Ser Glu Phe Ile Ser
   260 265 270
   Asn Thr Phe Leu Asp Lys Gln His Glu Val Glu Ile Pro Ser Pro Thr
   275 280 285
   Gln Lys Glu Lys Glu Lys Lys Lys Arg Pro Met Ser Gln Ile Ser Gly
   290 295 300
   Val Lys Lys Leu Met His Ser Ser Ser Leu Thr Asn Ser Ser Ile Pro
   305 310 315 320
   Arg Phe Gly Val Lys Thr Glu Gln Glu Asp Val Leu Ala Lys Glu Leu
   325 330 335
   Glu Asp Val Asn Lys Trp Gly Leu His Val Phe Arg Ile Ala Glu Leu
   340 345 350
   Ser Gly Asn Arg Pro Leu Thr Val Ile Met His Thr Ile Phe Gln Glu
   355 360 365
   Arg Asp Leu Leu Lys Thr Phe Lys Ile Pro Val Asp Thr Leu Ile Thr
   370 375 380
   Tyr Leu Met Thr Leu Glu Asp His Tyr His Ala Asp Val Ala Tyr His
   385 390 395 400
   Asn Asn Ile His Ala Ala Asp Val Val Gln Ser Thr His Val Leu Leu
   405 410 415
   Ser Thr Pro Ala Leu Glu Ala Val Phe Thr Asp Leu Glu Ile Leu Ala
   420 425 430
   Ala Ile Phe Ala Ser Ala Ile His Asp Val Asp His Pro Gly Val Ser
   435 440 445
   Asn Gln Phe Leu Ile Asn Thr Asn Ser Glu Leu Ala Leu Met Tyr Asn
   450 455 460
   Asp Ser Ser Val Leu Glu Asn His His Leu Ala Val Gly Phe Lys Leu
   465 470 475 480
   Leu Gln Glu Glu Asn Cys Asp Ile Phe Gln Asn Leu Thr Lys Lys Gln
   485 490 495
   Arg Gln Ser Leu Arg Lys Met Val Ile Asp Ile Val Leu Ala Thr Asp
   500 505 510
   Met Ser Lys His Met Asn Leu Leu Ala Asp Leu Lys Thr Met Val Glu
   515 520 525
   Thr Lys Lys Val Thr Ser Ser Gly Val Leu Leu Leu Asp Asn Tyr Ser
   530 535 540
   Asp Arg Ile Gln Val Leu Gln Asn Met Val His Cys Ala Asp Leu Ser
   545 550 555 560
   Asn Pro Thr Lys Pro Leu Gln Leu Tyr Arg Gln Trp Thr Asp Arg Ile
   565 570 575
   Met Glu Glu Phe Phe Arg Gln Gly Asp Arg Glu Arg Glu Arg Gly Met
   580 585 590
   Glu Ile Ser Pro Met Cys Asp Lys His Asn Ala Ser Val Glu Lys Ser
   595 600 605
   Gln Val Gly Phe Ile Asp Tyr Ile Val His Pro Leu Trp Glu Thr Trp
   610 615 620
   Ala Asp Leu Val His Pro Asp Ala Gln Asp Ile Leu Asp Thr Leu Glu
   625 630 635 640
   Asp Asn Arg Glu Trp Tyr Gln Ser Thr Ile Pro Gln Ser Pro Ser Pro
   645 650 655
   Ala Pro Asp Asp Pro Glu Glu Gly Arg Gln Gly Gln Thr Glu Lys Phe
   660 665 670
   Gln Phe Glu Leu Thr Leu Glu Glu Asp Gly Glu Ser Asp Thr Glu Lys
   675 680 685
   Asp Ser Gly Ser Gln Val Glu Glu Asp Thr Ser Cys Ser Asp Ser Lys
   690 695 700
   Thr Leu Cys Thr Gln Asp Ser Glu Ser Thr Glu Ile Pro Leu Asp Glu
   705 710 715 720
   Gln Val Glu Glu Glu Ala Val Gly Glu Glu Glu Glu Ser Gln Pro Glu
   725 730 735
   Ala Cys Val Ile Asp Asp Arg Ser Pro Asp Thr
   740 745
<210> 4
<211> 689
<212> PRT
<213> Homo sapiens
<220>
<221> PDE4D6
<222> (1)..(689)
<223>
<220>
<221> isoform_specific_N-terminus
<222> (1)..(32)
<223>
<220>
<221> LF1_splice_site
<222> (33)..(33)
<223>
<220>
<221> UCR1_start
<222> (66)..(66)
<223>
<220>
<221> UCR1_start-13
<222> (53)..(53)
<223>
<220>
<221> Ser54
<222> (70)..(70)
<223>
<220>
<221> Ser579
<222> (595)..(595)
<223>
<400> 4
   Met Ala Phe Val Trp Asp Pro Leu Gly Ala Thr Val Pro Gly Pro Ser
   1 5 10 15
   Thr Arg Ala Lys Ser Arg Leu Arg Phe Ser Lys Ser Tyr Ser Lys Leu
   20 25 30
   Phe Asp Val Asp Asn Gly Thr Ser Ala Gly Arg Ser Pro Leu Asp Pro
   35 40 45
   Met Thr Ser Pro Gly Ser Gly Leu Ile Leu Gln Ala Asn Phe Val His
   50 55 60
   Ser Gln Arg Arg Glu Ser Phe Leu Tyr Arg Ser Asp Ser Asp Tyr Asp
   65 70 75 80
   Leu Ser Pro Lys Ser Met Ser Arg Asn Ser Ser Ile Ala Ser Asp Ile
   85 90 95
   His Gly Asp Asp Leu Ile Val Thr Pro Phe Ala Gln Val Leu Ala Ser
   100 105 110
   Leu Arg Thr Val Arg Asn Asn Phe Ala Ala Leu Thr Asn Leu Gln Asp
   115 120 125
   Arg Ala Pro Ser Lys Arg Ser Pro Met Cys Asn Gln Pro Ser Ile Asn
   130 135 140
   Lys Ala Thr Ile Thr Glu Glu Ala Tyr Gln Lys Leu Ala Ser Glu Thr
   145 150 155 160
   Leu Glu Glu Leu Asp Trp Cys Leu Asp Gln Leu Glu Thr Leu Gln Thr
   165 170 175
   Arg His Ser Val Ser Glu Met Ala Ser Asn Lys Phe Lys Arg Met Leu
   180 185 190
   Asn Arg Glu Leu Thr His Leu Ser Glu Met Ser Arg Ser Gly Asn Gln
   195 200 205
   Val Ser Glu Phe Ile Ser Asn Thr Phe Leu Asp Lys Gln His Glu Val
   210 215 220
   Glu Ile Pro Ser Pro Thr Gln Lys Glu Lys Glu Lys Lys Lys Arg Pro
   225 230 235 240
   Met Ser Gln Ile Ser Gly Val Lys Lys Leu Met His Ser Ser Ser Leu
   245 250 255
   Thr Asn Ser Ser Ile Pro Arg Phe Gly Val Lys Thr Glu Gln Glu Asp
   260 265 270
   Val Leu Ala Lys Glu Leu Glu Asp Val Asn Lys Trp Gly Leu His Val
   275 280 285
   Phe Arg Ile Ala Glu Leu Ser Gly Asn Arg Pro Leu Thr Val Ile Met
   290 295 300
   His Thr Ile Phe Gln Glu Arg Asp Leu Leu Lys Thr Phe Lys Ile Pro
   305 310 315 320
   Val Asp Thr Leu Ile Thr Tyr Leu Met Thr Leu Glu Asp His Tyr His
   325 330 335
   Ala Asp Val Ala Tyr His Asn Asn Ile His Ala Ala Asp Val Val Gln
   340 345 350
   Ser Thr His Val Leu Leu Ser Thr Pro Ala Leu Glu Ala Val Phe Thr
   355 360 365
   Asp Leu Glu Ile Leu Ala Ala Ile Phe Ala Ser Ala Ile His Asp Val
   370 375 380
   Asp His Pro Gly Val Ser Asn Gln Phe Leu Ile Asn Thr Asn Ser Glu
   385 390 395 400
   Leu Ala Leu Met Tyr Asn Asp Ser Ser Val Leu Glu Asn His His Leu
   405 410 415
   Ala Val Gly Phe Lys Leu Leu Gln Glu Glu Asn Cys Asp Ile Phe Gln
   420 425 430
   Asn Leu Thr Lys Lys Gln Arg Gln Ser Leu Arg Lys Met Val Ile Asp
   435 440 445
   Ile Val Leu Ala Thr Asp Met Ser Lys His Met Asn Leu Leu Ala Asp
   450 455 460
   Leu Lys Thr Met Val Glu Thr Lys Lys Val Thr Ser Ser Gly Val Leu
   465 470 475 480
   Leu Leu Asp Asn Tyr Ser Asp Arg Ile Gln Val Leu Gln Asn Met Val
   485 490 495
   His Cys Ala Asp Leu Ser Asn Pro Thr Lys Pro Leu Gln Leu Tyr Arg
   500 505 510
   Gln Trp Thr Asp Arg Ile Met Glu Glu Phe Phe Arg Gln Gly Asp Arg
   515 520 525
   Glu Arg Glu Arg Gly Met Glu Ile Ser Pro Met Cys Asp Lys His Asn
   530 535 540
   Ala Ser Val Glu Lys Ser Gln Val Gly Phe Ile Asp Tyr Ile Val His
   545 550 555 560
   Pro Leu Trp Glu Thr Trp Ala Asp Leu Val His Pro Asp Ala Gln Asp
   565 570 575
   Ile Leu Asp Thr Leu Glu Asp Asn Arg Glu Trp Tyr Gln Ser Thr Ile
   580 585 590
   Pro Gln Ser Pro Ser Pro Ala Pro Asp Asp Pro Glu Glu Gly Arg Gln
   595 600 605
   Gly Gln Thr Glu Lys Phe Gln Phe Glu Leu Thr Leu Glu Glu Asp Gly
   610 615 620
   Glu Ser Asp Thr Glu Lys Asp Ser Gly Ser Gln Val Glu Glu Asp Thr
   625 630 635 640
   Ser Cys Ser Asp Ser Lys Thr Leu Cys Thr Gln Asp Ser Glu Ser Thr
   645 650 655
   Glu Ile Pro Leu Asp Glu Gln Val Glu Glu Glu Ala Val Gly Glu Glu
   660 665 670
   Glu Glu Ser Gln Pro Glu Ala Cys Val Ile Asp Asp Arg Ser Pro Asp
   675 680 685
   Thr
<210> 5
<211> 750
<212> PRT
<213> Homo sapiens
<220>
<221> PDE4D7
<222> (1)..(750)
<223>
<220>
<221> isoform_specific_N-terminus
<222> (1)..(93)
<223>
<220>
<221> LF1_splice_site
<222> (94)..(94)
<223>
<220>
<221> UCR1_start
<222> (127)..(127)
<223>
<220>
<221> UCR1_start-13
<222> (114)..(114)
<223>
<220>
<221> Ser54
<222> (131)..(131)
<223>
<220>
<221> Ser579
<222> (656)..(656)
<223>
<400> 5
   Met Lys Arg Asn Thr Cys Asp Leu Leu Ser Arg Ser Lys Ser Ala Ser
   1 5 10 15
   Glu Glu Thr Leu His Ser Ser Asn Glu Glu Glu Asp Pro Phe Arg Gly
   20 25 30
   Met Glu Pro Tyr Leu Val Arg Arg Leu Ser Cys Arg Asn Ile Gln Leu
   35 40 45
   Pro Pro Leu Ala Phe Arg Gln Leu Glu Gln Ala Asp Leu Lys Ser Glu
   50 55 60
   Ser Glu Asn Ile Gln Arg Pro Thr Ser Leu Pro Leu Lys Ile Leu Pro
   65 70 75 80
   Leu Ile Ala Ile Thr Ser Ala Glu Ser Ser Gly Lys Leu Phe Asp Val
   85 90 95
   Asp Asn Gly Thr Ser Ala Gly Arg Ser Pro Leu Asp Pro Met Thr Ser
   100 105 110
   Pro Gly Ser Gly Leu Ile Leu Gln Ala Asn Phe Val His Ser Gln Arg
   115 120 125
   Arg Glu Ser Phe Leu Tyr Arg Ser Asp Ser Asp Tyr Asp Leu Ser Pro
   130 135 140
   Lys Ser Met Ser Arg Asn Ser Ser Ile Ala Ser Asp Ile His Gly Asp
   145 150 155 160
   Asp Leu Ile Val Thr Pro Phe Ala Gln Val Leu Ala Ser Leu Arg Thr
   165 170 175
   Val Arg Asn Asn Phe Ala Ala Leu Thr Asn Leu Gln Asp Arg Ala Pro
   180 185 190
   Ser Lys Arg Ser Pro Met Cys Asn Gln Pro Ser Ile Asn Lys Ala Thr
   195 200 205
   Ile Thr Glu Glu Ala Tyr Gln Lys Leu Ala Ser Glu Thr Leu Glu Glu
   210 215 220
   Leu Asp Trp Cys Leu Asp Gln Leu Glu Thr Leu Gln Thr Arg His Ser
   225 230 235 240
   Val Ser Glu Met Ala Ser Asn Lys Phe Lys Arg Met Leu Asn Arg Glu
   245 250 255
   Leu Thr His Leu Ser Glu Met Ser Arg Ser Gly Asn Gln Val Ser Glu
   260 265 270
   Phe Ile Ser Asn Thr Phe Leu Asp Lys Gln His Glu Val Glu Ile Pro
   275 280 285
   Ser Pro Thr Gln Lys Glu Lys Glu Lys Lys Lys Arg Pro Met Ser Gln
   290 295 300
   Ile Ser Gly Val Lys Lys Leu Met His Ser Ser Ser Leu Thr Asn Ser
   305 310 315 320
   Ser Ile Pro Arg Phe Gly Val Lys Thr Glu Gln Glu Asp Val Leu Ala
   325 330 335
   Lys Glu Leu Glu Asp Val Asn Lys Trp Gly Leu His Val Phe Arg Ile
   340 345 350
   Ala Glu Leu Ser Gly Asn Arg Pro Leu Thr Val Ile Met His Thr Ile
   355 360 365
   Phe Gln Glu Arg Asp Leu Leu Lys Thr Phe Lys Ile Pro Val Asp Thr
   370 375 380
   Leu Ile Thr Tyr Leu Met Thr Leu Glu Asp His Tyr His Ala Asp Val
   385 390 395 400
   Ala Tyr His Asn Asn Ile His Ala Ala Asp Val Val Gln Ser Thr His
   405 410 415
   Val Leu Leu Ser Thr Pro Ala Leu Glu Ala Val Phe Thr Asp Leu Glu
   420 425 430
   Ile Leu Ala Ala Ile Phe Ala Ser Ala Ile His Asp Val Asp His Pro
   435 440 445
   Gly Val Ser Asn Gln Phe Leu Ile Asn Thr Asn Ser Glu Leu Ala Leu
   450 455 460
   Met Tyr Asn Asp Ser Ser Val Leu Glu Asn His His Leu Ala Val Gly
   465 470 475 480
   Phe Lys Leu Leu Gln Glu Glu Asn Cys Asp Ile Phe Gln Asn Leu Thr
   485 490 495
   Lys Lys Gln Arg Gln Ser Leu Arg Lys Met Val Ile Asp Ile Val Leu
   500 505 510
   Ala Thr Asp Met Ser Lys His Met Asn Leu Leu Ala Asp Leu Lys Thr
   515 520 525
   Met Val Glu Thr Lys Lys Val Thr Ser Ser Gly Val Leu Leu Leu Asp
   530 535 540
   Asn Tyr Ser Asp Arg Ile Gln Val Leu Gln Asn Met Val His Cys Ala
   545 550 555 560
   Asp Leu Ser Asn Pro Thr Lys Pro Leu Gln Leu Tyr Arg Gln Trp Thr
   565 570 575
   Asp Arg Ile Met Glu Glu Phe Phe Arg Gln Gly Asp Arg Glu Arg Glu
   580 585 590
   Arg Gly Met Glu Ile Ser Pro Met Cys Asp Lys His Asn Ala Ser Val
   595 600 605
   Glu Lys Ser Gln Val Gly Phe Ile Asp Tyr Ile Val His Pro Leu Trp
   610 615 620
   Glu Thr Trp Ala Asp Leu Val His Pro Asp Ala Gln Asp Ile Leu Asp
   625 630 635 640
   Thr Leu Glu Asp Asn Arg Glu Trp Tyr Gln Ser Thr Ile Pro Gln Ser
   645 650 655
   Pro Ser Pro Ala Pro Asp Asp Pro Glu Glu Gly Arg Gln Gly Gln Thr
   660 665 670
   Glu Lys Phe Gln Phe Glu Leu Thr Leu Glu Glu Asp Gly Glu Ser Asp
   675 680 685
   Thr Glu Lys Asp Ser Gly Ser Gln Val Glu Glu Asp Thr Ser Cys Ser
   690 695 700
   Asp Ser Lys Thr Leu Cys Thr Gln Asp Ser Glu Ser Thr Glu Ile Pro
   705 710 715 720
   Leu Asp Glu Gln Val Glu Glu Glu Ala Val Gly Glu Glu Glu Glu Ser
   725 730 735
   Gln Pro Glu Ala Cys Val Ile Asp Asp Arg Ser Pro Asp Thr
   740 745 750
<210> 6
<211> 679
<212> PRT
<213> Homo sapiens
<220>
<221> PDE4D8
<222> (1)..(679)
<223>
<220>
<221> isoform_specific_N-terminus
<222> (1)..(23)
<223>
<220>
<221> LF1_splice_site
<222> (23)..(23)
<223>
<220>
<221> UCR1_start
<222> (56)..(56)
<223>
<220>
<221> UCR1_start-13
<222> (43)..(43)
<223>
<220>
<221> Ser54
<222> (60)..(60)
<223>
<220>
<221> Ser579
<222> (585)..(585)
<223>
<400> 6
   Met Ser Ile Ile Met Lys Pro Arg Ser Arg Ser Thr Ser Ser Leu Arg
   1 5 10 15
   Thr Ala Glu Ala Lys Leu Phe Asp Val Asp Asn Gly Thr Ser Ala Gly
   20 25 30
   Arg Ser Pro Leu Asp Pro Met Thr Ser Pro Gly Ser Gly Leu Ile Leu
   35 40 45
   Gln Ala Asn Phe Val His Ser Gln Arg Arg Glu Ser Phe Leu Tyr Arg
   50 55 60
   Ser Asp Ser Asp Tyr Asp Leu Ser Pro Lys Ser Met Ser Arg Asn Ser
   65 70 75 80
   Ser Ile Ala Ser Asp Ile His Gly Asp Asp Leu Ile Val Thr Pro Phe
   85 90 95
   Ala Gln Val Leu Ala Ser Leu Arg Thr Val Arg Asn Asn Phe Ala Ala
   100 105 110
   Leu Thr Asn Leu Gln Asp Arg Ala Pro Ser Lys Arg Ser Pro Met Cys
   115 120 125
   Asn Gln Pro Ser Ile Asn Lys Ala Thr Ile Thr Glu Glu Ala Tyr Gln
   130 135 140
   Lys Leu Ala Ser Glu Thr Leu Glu Glu Leu Asp Trp Cys Leu Asp Gln
   145 150 155 160
   Leu Glu Thr Leu Gln Thr Arg His Ser Val Ser Glu Met Ala Ser Asn
   165 170 175
   Lys Phe Lys Arg Met Leu Asn Arg Glu Leu Thr His Leu Ser Glu Met
   180 185 190
   Ser Arg Ser Gly Asn Gln Val Ser Glu Phe Ile Ser Asn Thr Phe Leu
   195 200 205
   Asp Lys Gln His Glu Val Glu Ile Pro Ser Pro Thr Gln Lys Glu Lys
   210 215 220
   Glu Lys Lys Lys Arg Pro Met Ser Gln Ile Ser Gly Val Lys Lys Leu
   225 230 235 240
   Met His Ser Ser Ser Leu Thr Asn Ser Ser Ile Pro Arg Phe Gly Val
   245 250 255
   Lys Thr Glu Gln Glu Asp Val Leu Ala Lys Glu Leu Glu Asp Val Asn
   260 265 270
   Lys Trp Gly Leu His Val Phe Arg Ile Ala Glu Leu Ser Gly Asn Arg
   275 280 285
   Pro Leu Thr Val Ile Met His Thr Ile Phe Gln Glu Arg Asp Leu Leu
   290 295 300
   Lys Thr Phe Lys Ile Pro Val Asp Thr Leu Ile Thr Tyr Leu Met Thr
   305 310 315 320
   Leu Glu Asp His Tyr His Ala Asp Val Ala Tyr His Asn Asn Ile His
   325 330 335
   Ala Ala Asp Val Val Gln Ser Thr His Val Leu Leu Ser Thr Pro Ala
   340 345 350
   Leu Glu Ala Val Phe Thr Asp Leu Glu Ile Leu Ala Ala Ile Phe Ala
   355 360 365
   Ser Ala Ile His Asp Val Asp His Pro Gly Val Ser Asn Gln Phe Leu
   370 375 380
   Ile Asn Thr Asn Ser Glu Leu Ala Leu Met Tyr Asn Asp Ser Ser Val
   385 390 395 400
   Leu Glu Asn His His Leu Ala Val Gly Phe Lys Leu Leu Gln Glu Glu
   405 410 415
   Asn Cys Asp Ile Phe Gln Asn Leu Thr Lys Lys Gln Arg Gln Ser Leu
   420 425 430
   Arg Lys Met Val Ile Asp Ile Val Leu Ala Thr Asp Met Ser Lys His
   435 440 445
   Met Asn Leu Leu Ala Asp Leu Lys Thr Met Val Glu Thr Lys Lys Val
   450 455 460
   Thr Ser Ser Gly Val Leu Leu Leu Asp Asn Tyr Ser Asp Arg Ile Gln
   465 470 475 480
   Val Leu Gln Asn Met Val His Cys Ala Asp Leu Ser Asn Pro Thr Lys
   485 490 495
   Pro Leu Gln Leu Tyr Arg Gln Trp Thr Asp Arg Ile Met Glu Glu Phe
   500 505 510
   Phe Arg Gln Gly Asp Arg Glu Arg Glu Arg Gly Met Glu Ile Ser Pro
   515 520 525
   Met Cys Asp Lys His Asn Ala Ser Val Glu Lys Ser Gln Val Gly Phe
   530 535 540
   Ile Asp Tyr Ile Val His Pro Leu Trp Glu Thr Trp Ala Asp Leu Val
   545 550 555 560
   His Pro Asp Ala Gln Asp Ile Leu Asp Thr Leu Glu Asp Asn Arg Glu
   565 570 575
   Trp Tyr Gln Ser Thr Ile Pro Gln Ser Pro Ser Pro Ala Pro Asp Asp
   580 585 590
   Pro Glu Glu Gly Arg Gln Gly Gln Thr Glu Lys Phe Gln Phe Glu Leu
   595 600 605
   Thr Leu Glu Glu Asp Gly Glu Ser Asp Thr Glu Lys Asp Ser Gly Ser
   610 615 620
   Gln Val Glu Glu Asp Thr Ser Cys Ser Asp Ser Lys Thr Leu Cys Thr
   625 630 635 640
   Gln Asp Ser Glu Ser Thr Glu Ile Pro Leu Asp Glu Gln Val Glu Glu
   645 650 655
   Glu Ala Val Gly Glu Glu Glu Glu Ser Gln Pro Glu Ala Cys Val Ile
   660 665 670
   Asp Asp Arg Ser Pro Asp Thr
   675
<210> 7
<211> 809
<212> PRT
<213> Homo sapiens
<220>
<221> PDE4D4
<222> (1)..(809)
<223>
<220>
<221> LF1_splice_site
<222> (153)..(153)
<223>
<220>
<221> UCR_1_start
<222> (186)..(186)
<223>
<220>
<221> UCR1_start_-13
<222> (173)..(173)
<223>
<220>
<221> Ser54
<222> (190)..(190)
<223>
<220>
<221> Ser579
<222> (715)..(715)
<223>
<400> 7
<210> 8
<211> 1974
<212> DNA
<213> Homo sapiens
<220>
<221> PDE4D_core_contruct
<222> (1)..(1974)
<223>
<400> 8
   tttgatgtgg acaatggcac atctgcggga cggagtccct tggatcccat gaccagccca      60
   ggatccgggc taattctcca agcaaatttt gtccacagtc aacgacggga gtccttcctg     120
   tatcgatccg acagcgatta tgacctctct ccaaagtcta tgtcccggaa ctcctccatt     180
   gccagtgata tacacggaga tgacttgatt gtgactccat ttgctcaggt cttggccagt     240
   ctgcgaactg tacgaaacaa ctttgctgca ttaactaatt tgcaagatcg agcacctagc     300
   aaaagatcac ccatgtgcaa ccaaccatcc atcaacaaag ccaccataac agaggaggcc     360
   taccagaaac tggccagcga gaccctggag gagctggact ggtgtctgga ccagctagag     420
   accctacaga ccaggcactc cgtcagtgag atggcctcca acaagtttaa aaggatgctt     480
   aatcgggagc tcacccatct ctctgaaatg agtcggtctg gaaatcaagt gtcagagttt     540
   atatcaaaca cattcttaga taagcaacat gaagtggaaa ttccttctcc aactcagaag     600
   gaaaaggaga aaaagaaaag accaatgtct cagatcagtg gagtcaagaa attgatgcac    660
   agctctagtc tgactaattc aagtatccca aggtttggag ttaaaactga acaagaagat    720
   gtccttgcca aggaactaga agatgtgaac aaatggggtc ttcatgtttt cagaatagca    780
   gagttgtctg gtaaccggcc cttgactgtt atcatgcaca ccatttttca ggaacgggat    840
   ttattaaaaa catttaaaat tccagtagat actttaatta catatcttat gactctcgaa    900
   gaccattacc atgctgatgt ggcctatcac aacaatatcc atgctgcaga tgttgtccag    960
   tctactcatg tgctattatc tacacctgct ttggaggctg tgtttacaga tttggagatt   1020
   cttgcagcaa tttttgccag tgcaatacat gatgtagatc atcctggtgt gtccaatcaa   1080
   tttctgatca atacaaactc tgaacttgcc ttgatgtaca atgattcctc agtcttagag   1140
   aaccatcatt tggctgtggg ctttaaattg cttcaggaag aaaactgtga cattttccag   1200
   aatttgacca aaaaacaaag acaatcttta aggaaaatgg tcattgacat cgtacttgca   1260
   acagatatgt caaaacacat gaatctactg gctgatttga agactatggt tgaaactaag   1320
   aaagtgacaa gctctggagt tcttcttctt gataattatt ccgataggat tcaggttctt   1380
   cagaatatgg tgcactgtgc agatctgagc aacccaacaa agcctctcca gctgtaccgc   1440
   cagtggacgg accggataat ggaggagttc ttccgccaag gagaccgaga gagggaacgt   1500
   ggcatggaga taagccccat gtgtgacaag cacaatgctt ccgtggaaaa atcacaggtg   1560
   ggcttcatag actatattgt tcatcccctc tgggagacat gggcagacct cgtccaccct   1620
   gacgcccagg atattttgga cactttggag gacaatcgtg aatggtacca gagcacaatc   1680
   cctcagagcc cctctcctgc acctgatgac ccagaggagg gccggcaggg tcaaactgag   1740
   aaattccagt ttgaactaac tttagaggaa gatggtgagt cagacacgga aaaggacagt   1800
   ggcagtcaag tggaagaaga cactagctgc agtgactcca agactctttg tactcaagac   1860
   tcagagtcta ctgaaattcc ccttgatgaa caggttgaag aggaggcagt aggggaagaa   1920
   gaggaaagcc agcctgaagc ctgtgtcata gatgatcgtt ctcctgacac gtaa         1974

## Description

The invention relates to modified phosphodiesterase (PDE) polypeptides with altered physicochemical properties which are particularly useful in screening assays for the identification of agonists or antagonists of PDEs, the polynucleotides encoding such modified PDE polypeptides, antibodies against such polypeptides, methods of preparation and uses of such polypeptides.

The phosphodiesterases (PDEs) represent a family of enzymes that catalyze the hydrolysis of the various cyclic nucleoside monophosphates (including cAMP). These cyclic nucleotides have been found to act as second messengers within the cells by carrying impulses from cell surface receptors having bound various hormones and neurotransmitters to the inside of the cells. The task of phosphodiesterases is to degrade these cyclic mononucleotides once their messenger role is completed thereby regulating the level of cyclic nucleotides within the cells and maintaining cyclic nucleotide homeostasis.

Ten families of PDEs have been identified, designated PDE1 to PDE10. Within each family there are two or more related but distinct gene products (A, B, C, etc.) and for each of these alternative mRNA processing gives rise to multiple splice variants, identified by an additional arabic numeral in accordance with the most recent nomenclature recommendation (Molecular Pharmacology 46, 399-405, 1994).

All PDE gene products identified so far have one catalytic and a least one regulatory domain. The catalytic domain lies towards the carboxylic acid terminus of each PDE protein and has the greatest homology between the PDE families, being >75% homologous at the amino acid level (Perry and Higgs, Curr. Opinion Chem. Biol. 2, 472-481, 1998). Nevertheless, each of the more than 30 known PDEs have individually distinct substrate specificities, kinetic characteristics, regulatory properties and cellular and subcellular distributions (Houslay and Milligan, Trends in Biochem. Sci. 22, 217-224, 1997).

PDEs 4, 7 and 8 are highly specific for cAMP. PDEs 5, 6, 9 and 10 are selective for cGMP. PDE3s bind cAMP and cGMP with similar affinity, but hydrolyse cAMP most efficiently, cGMP rather poorly. PDE3s are, therefore, negatively regulated in their cAMP hydrolysing ability by cGMP. PDEs 1 and 2 hydrolyse both cAMP and cGMP, but with PDE1 the relative efficiencies vary with isoenzyme subtype (Perry and Higgs, Curr. Opinion Chem. Biol. 2,472-481,1998).

The amino terminal ends of PDEs consist of the regulatory domains, which are very different both between families and between variants within families. This region contains variously: a binding domain for Ca2+-calmodulin (CaM) in PDE1 ; non-catalytic cGMP-35 binding sites in PDEs 2, 5 and 6; a binding domain for the signalling G-protein transducin in PDE6. The amino terminal region also contains protein- and membrane-targeting sequences in several PDE3s and PDE4s, as well as protein kinase phosphorylation sites in PDEs 1, 3, 4 and 5. These phosphorylation sites are likely to be important in regulation of catalytic activity and/or subcellular location (Perry and Higgs, Curr. Opinion Chem. Biol. 2, 472-481, 1998).

Of particular interest to this invention are the PDE4 enzymes. PDE4 enzymes share a common structure, as deduced from their amino acid sequences (Beavo and Reifsnyder, Trends Pharmacol. Sci. 11, 150-155, 1990; Bolger at al., Mol. Cell Biol. 13, 6558-6571, 1993; Houslay, Sullivan and Bolger, Adv. Pharmacol. 44, 225-242, 1998). Members of each gene family (PDE4A, PDE4B, PDE4C, PDE4D) share common C-terminal regions, different for each family, and catalytic domains that for all PDE4 isoforms are very similar (84% homology over about 360 amino acids across all PDE4s; Houslay, Sullivan and Bolger, Adv. Pharmacol. 44, 225-242, 1998).

From N-terminus to catalytic region, the sequence in "long form" PDE4s can be divided into 5 regions, three of which are isoform-specific (N-terminal region, linker regions 1 and 2, or LR1 and LR2) and two, more conserved regions, that are broadly similar between all isoforms, the upstream conserved regions 1 and 2 (UCR1 and UCR2). "Short form" PDE4s, e.g. PDE4A1, PDE4B2, PDE4D1, PDE4D2, lack UCR1 and LR1 plus differing amounts of the N-terminal region of UCR2. Throughout all regions are potential phosphorylation sites for a variety of kinases, including PKA (e.g. Ser 54 in human PDE4D3), mitogen activated protein kinases (e.g. Ser 487 of human PDE4B2), casein kinase II (e.g. Ser 489 of PDE4B2) and calcium-diacylglycerol dependent protein kinases (Houslay, Sullivan and Bolger, Adv. Pharmacol. 44, 225-242, 1998). Phosphorylations at some of these sites have been shown to activate the PDEs (e.g. Ser 54), others serve to inhibit. There is also evidence that some phosphorylations serve to prime the enzymes ready for subsequent activation by further phosphorylation at a different site or sites (Houslay, Sullivan and Bolger, Adv. Pharmacol. 44, 225-242, 1998). Other auto-regulatory sites may be found in the N-terminal sequence of certain PDE4s (Bolger at al., Mol. Cell Biol. 13, 6558-6571, 1993, Mc Phee et al., Biochem. J. 310, 965-974,1995).

Specific PDE4 inhibition may be useful over a very wide range of disease areas. These include: asthma, atopic dermatitis, depression, reperfusion injury, septic shock, toxic shock, autoimmune diabetes, AIDS, Crohn's disease, multiple sclerosis, cerebral ischemia, psoriasis, allograft rejection, restenosis, stroke, ulcerative colitis, cachexia, cerebral malaria, allergic rhinoconjunctivitis, osteoarthritis, rheutmatoid arthritis, autoimmune encephalomyelitis (Houslay, Sullivan and Bolger, Adv. Pharmacol. 44, 225-242, 1998, WO02/074992). In the area of asthma, PDE4 inhibition helps to increase cAMP in bronchial smooth muscle, thereby producing a modest bronchodilatory effect, of use in the alleviation of asthmatic symptoms. But perhaps most importantly, inhibition of PDE4s is now a recognised method by which to suppress immune and inflammatory cell responses (Hughes et al. Drug Discov. Today 2, 89-101, 1997; Torphy, Am. J.Respir. Crit. Care Med. 157, 351-370, 1998; Teixeira et al., Trends Pharm. Sci. 18, 164-171,1997).

PDE4s play major roles in modulating the activity of virtually every cell type involved in the inflammatory process. Immune and inflammatory conditions occur when recruitment of leukocytes from the blood compartment into tissues is either uncontrolled, inappropriate, prolonged or directed against self. In asthma, rheumatoid arthritis and multiple sclerosis, infiltration of tissues with inflammatory cells is prolonged and intense, leading ultimately to severe (and self-perpetuating) damage and loss of function. Acute disregulation of the immune system occurs in such conditions as acute respiratory distress syndrome (ARDS) where an overwhelming and generalised inflammatory response can frequently lead to death. There is also substantial evidence which suggests that inflammation may play a part in defining the extent of injury resulting from reperfusion following ischaemia, at least in brain and lung (Entman and Smith, Cardiovasc. Res. 28, 1301-1311, 1994).

The PDE4D phosphodiesterases, which are the most preferred focus of the present invention, exist in mammals in the form of isoenzymes (which represent different molecular forms of the same enzyme polypeptide). These phosphodiesterases are localized in the cytosol of the cell and are unassociated with any known membraneous structures. The PDE4D isoenzymes specifically degrade cAMP and are a common target for such pharmacological agents as antidepressants (for example, rolipram). Several splice forms of PDE4D are known. The short forms, PDE4D1 and PDE4D2, lack UCR1 and LR1 sites. Rather than these, interest of the present invention focuses on the long isoforms, of which 6 are known, namely PDE4D3, PDE4D4, PDE4D5, PDE4D6, PDE4D7 and PDE4D8. All of these have in common the LR1 and UCR1 sites and the domains located at the C-terminus of these sites, but they have different N-terminal domains. Isoforms PDE4D6, D7 and D8 were recently disclosed in WO02/074992.

For identification of compounds that inhibit PDE4D isoenzymes, screening assays are used that are either whole cell assays in which the cells are treated with the inhibitor and cAMP hydrolysis is measured, or in vitro assays which use purified PDE4D isoenzymes either isolated from their natural sources or from cells overexpressing recombinant PDE4D isoenzymes. Such assays are described e.g. in WO00/09504, WO01/35979, WO01/68600 and WO98/45268. WO01/35979 describes the purification of recombinant PDE4D expressed in Saccharomyces Cerevisiae. The use of recombinant native forms of PDE4D for the identification of PDE agonists or antagonists has the disadvantage that the PDE4D poypeptide does not have optimal physicochemical characteristics, especially with respect to aggregate formation. Aggregate formation can lead to protein precipitation and is known to potentially decrease the activity of enzymes, compared to the authentic oligomeric molecules. Thus, aggregation of the native forms of PDE4D polypeptides in screening assays aimed at identifying agonists or antagonists of PDE4D phosphodiesterase activity negatively affects the quality of these assays. In addition, the use of assays based on individual recombinant PDE4D isoforms has the disadvantage of potentially resulting in the identification of agonists or antagonists that are specific for a single isoform of a PDE4D only, instead of agonists or antagonists which act on all long or short form PDE4D isoforms together.

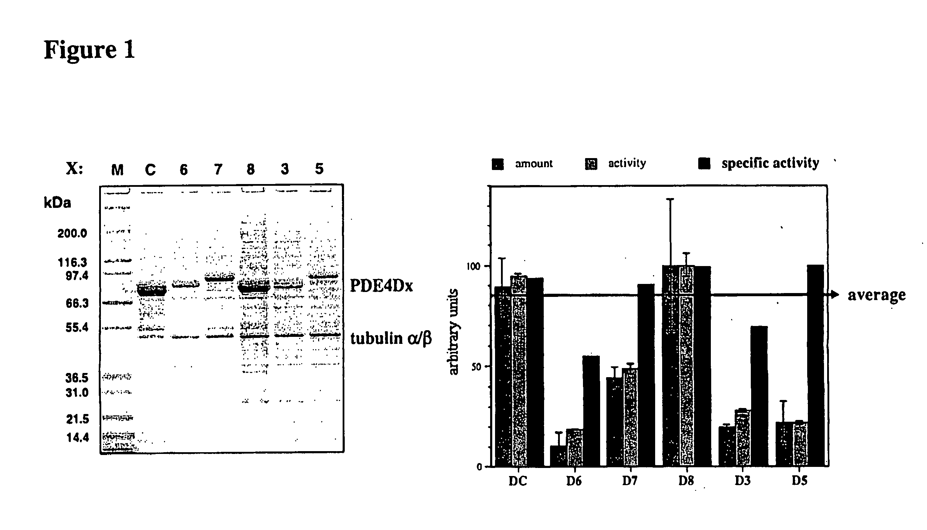

Figure 1: Specific phosphodiesterase activity for the different PDE4D isoforms D3 and D5 to D8, and the PDE4D core construct (DC).

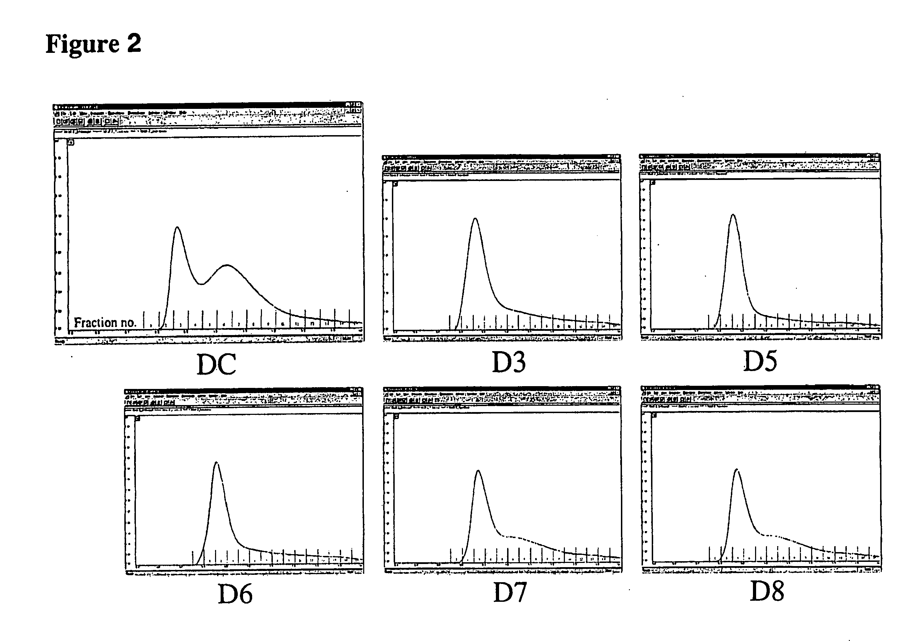

Figure 2: Aggregate formation by different PDE4D isoforms and the core construct. In this figure, the gel filtration profiles of different isoforms of PDE4D (D3, D5, D6, D7, D8), and the core construct (DC) are shown. The first elution peak represents PDE4D aggregates. The pronounced shoulder in the elution profile of DC, where polypeptides of approx. 300,000 Dalton elute, indicates a decrease in aggregation of DC, as compared to the five isoforms of PDE4D.

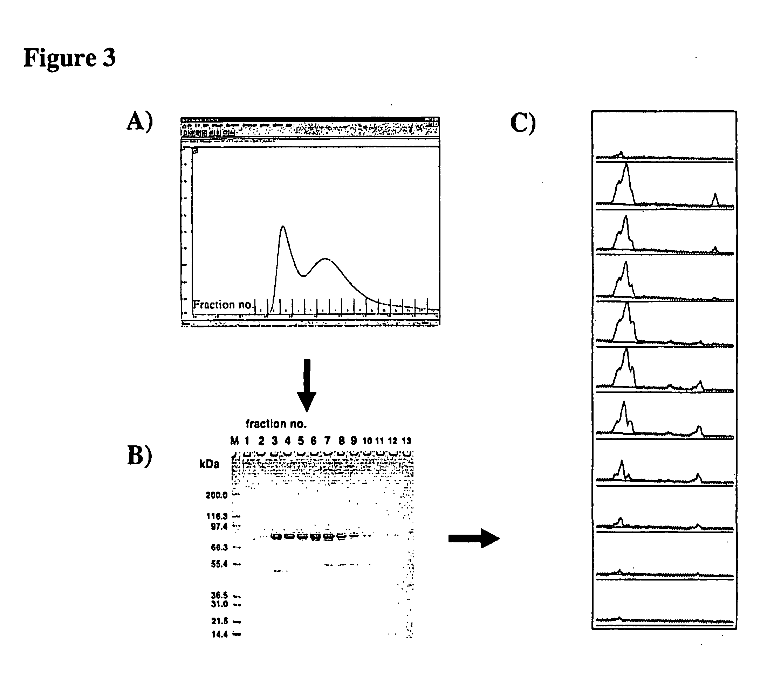

Figure 3: Quantitation of aggregation of the core construct (DC) of PDE4D, and of the tubulin content of the core construct perparations. In A), the elution profile of DC is shown, with the fractions indicated on the bottom. B) shows an SDS-PAGE of the different fractions from the gelfiltration. C) shows the analysis of optical densities of individual fractions corresponding to the Coomassie stained SDS-PAGE in B).

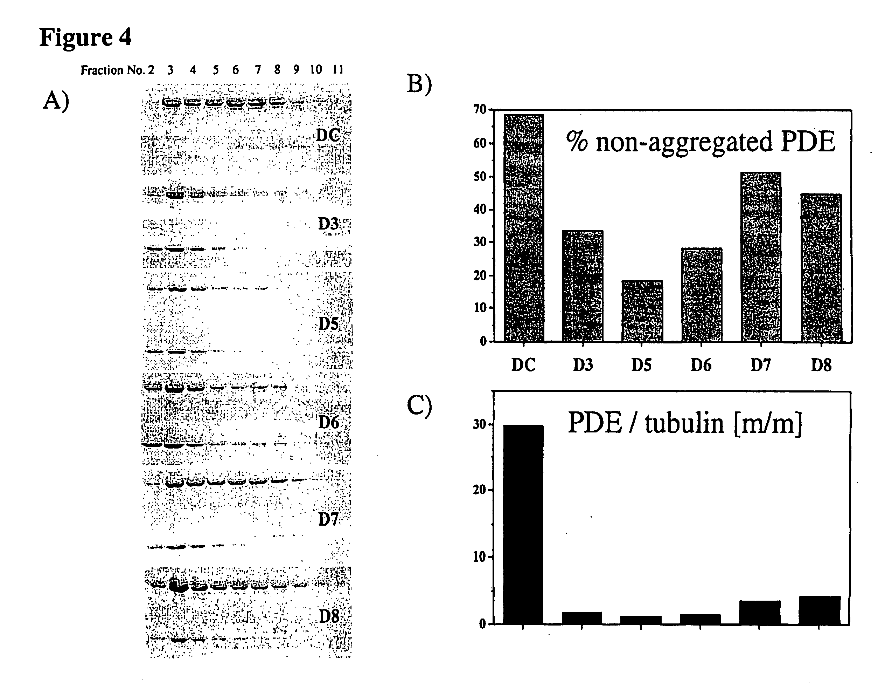

Figure 4: Differences in aggregation and tubulin content of different isoforms and the core construct of PDE. A) SDS-PAGE gels are shown for DC and five isoforms of PDE4D which were analysed as described above. B) The percentage of non-aggregated PDE4D is shown for the core construct and the isoforms D3 and D5 to D8, based on total PDE4D protein. DC has the highest percentage of non-aggregated PDE4D, as compared to the five isoforms. C) This figure shows the ratio between PDE4D and tubulin for DC and the isoforms D3 and D5 to D8, based on total PDE mass and total tubulin mass. As can be seen, the tubulin content of DC is significantly lower than that of the five PDE4D isoforms indicated.

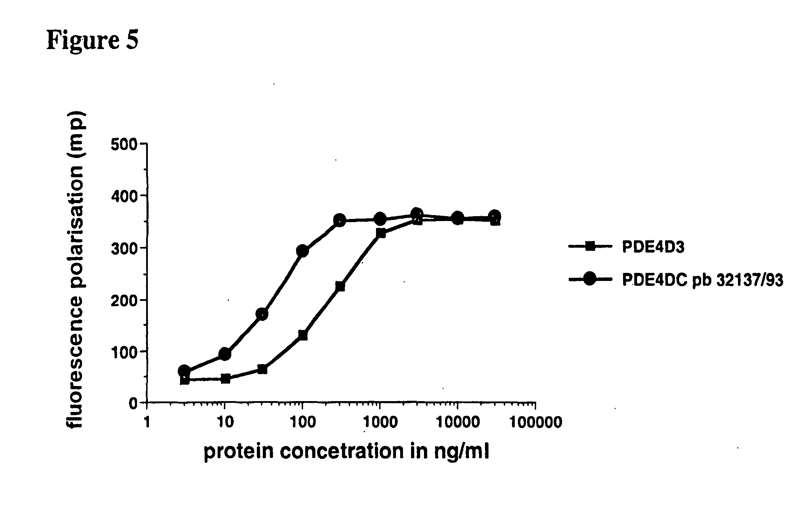

Figure 5: Activity assay. Phosphodiesterase activity is shown both for the PDE4D core construct (DC) and PDE4D3 (D3).

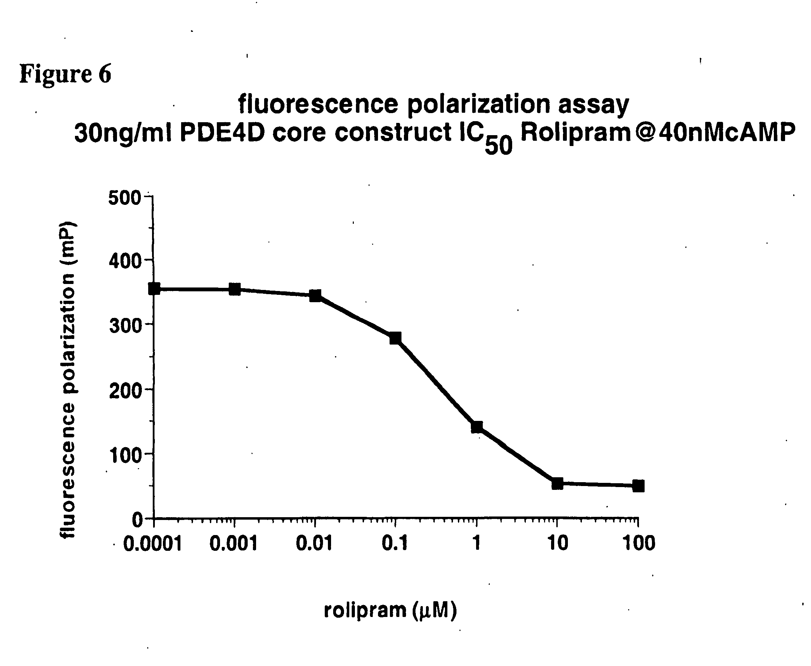

Figure 6: Rolipram inhibition. Phosphodiesterase activity of the PDE4D core construct (DC) can be inhibited by Rolipram. The IC50 of DC phosphodiesterase activity by Rolipram inhibition was 0.34+/- 0.06 mM.

The term "polypeptide" as used herein, refers to a polymer of amino acids, and not to a specific length. Thus, peptides, oligopeptides and proteins are included within the definition of polypeptide.

The term "physicochemical properties" as used herein refers to the propensity of polypeptides to monomer formation, oligomer formation or aggregate formation.

The terms "aggregation" and "aggregate formation" as used herein are used interchangeably and refer to the type of association of polypeptide chains which, in contrast to oligomer formation, is an irreversible process and does not follow the law of mass interaction. Aggregation progresses in a time dependent manner and does not stop when a defined number of chains have associated. Aggregation of polypeptides can lead to protein precipitation because the molecular size of the aggregate is so dramatically increased that the solubility of the molecules is impaired. Aggregation is also known to potentially decrease the activity of the enzyme as compared to authentic oligomeric molecules.

The term "oligomer formation" as used herein refers to a reversible association of polypeptide chains whereby the ratio of oligomeric proteins and monomeric proteins at a certain protein concentration and at thermodynamic equilibrium is described by the association constant.

The term "non-aggregated" refers to polypeptides that are present either as oligomers or monomers or a mixture of oligomers and monomers at equilibrium according to the law of mass interaction.

The term "improved physicochemical properties" as used herein refers to a decrease in aggregate formation of polypeptides. For the purpose of definition, aggregate formation of the polypeptides of this invention is determined by quantitation of the fractions of a gelfiltration experiment in which aggregated PDE elutes, and of the fractions in which non-aggregated PDE elutes. The degree of aggregation is calculated in percent of non-aggregated PDE based on total (aggregated and non-aggregated) PDE. For the purpose of the definition of aggregation, aggregated PDE is considered to elute in fractions 2 to 4, in which aggregated molecules of at least 2 Mio Daltons elute, and non-aggregated PDE is considered to elute in fractions 5 to 11, corresponding to the fractions in which molecules of about 0.3 Mio Daltons elute, when the following conditions are used for gel filtration: 50 µl of Ni-NTA agarose purified isoform preparation are injected into a Superose 12 size exclusion column (type PC3.2/30; Amersham Pharmacia Biotech), equilibrated with 50 mM TrisHCl pH 7.7, 100 mM NaCl, 0.5 mM MgCl2 at a flow rate of 0.1 ml/min at 4°C. Chromatograms are recorded at 278 nm. Starting from the elution volume onwards, the column eluate is collected as 50 µl fractions.

The term "long form PDE" as used herein refers to PDE isoforms which comprise an isoform specific N-terminal domain, linker regions LR1 and/or LR2, and upstream conserved regions UCR1 and UCR2.

The term "UCR1 start" as used herein refers to the first amino acid of the UCR1 domain of PDE4D, as annotated in SEQ ID No. 1 to 7.

The term "LF1 site" as used herein refers to the first amino acid of the splice site of PDE4D as indicated in SEQ ID No. 1-7.

The term "amino acids upstream of LF1" refers to the position of an amino acid counted from the LF-1 site towards the amino terminus of the native protein.

The term "D3" as used herein refers to the PDE4D3 isoform. The term "D4" as used herein refers to the PDE4D4 isoform. The term "D5" as used herein refers to the PDE4D5 isoform. The term "D6" as used herein refers to the PDE4D6 isoform as provided by WO02/074992. The term "D7" as used herein refers to the PDE4D7 isoform as provided by WO02/074992. The term "D8" as used herein refers to the PDE4D8 isoform as provided by WO02/074992. The polypeptide sequences of the human PDE4D isoforms D3, D4, D5, D6, D7, and D8 are shown in SEQ ID No.1 and 3-7.

The term "Ser54" as used herein refers to the Ser residue in position 54 of PDE4D3, and to the Ser residue in the respective position of any of the other PDE polypeptides. The position corresponding to Ser54 of PDE4D3 is annotated in SEQ ID No. 1 to 7 for the different isoforms and the core sequence which is common to all the PDE4D isoforms.

The term "Ser579" as used herein refers to the Ser residue in position 579 of PDE4D3, and to the Ser residue in the respective position of any of the other PDE polypeptides. The position corresponding to Ser579 of PDE4D3 is annotated in SEQ ID No. 1 to 7 for the different isoforms and the core sequence which is common to all the PDE4D isoforms.

The term "tubulin association" as used herein refers to the tubulin content of purified PDE preparations.

As used herein, the term "agonist" also refers to activators, and the term "antagonist" also refers to inhibitors of PDE phosphodiesterase activity.

The disadvantage of native recombinant PDE polypeptides resides in the formation of aggregates of the polypeptides, which gives rise to sub-optimal conditions for the identification of modulators of PDE phosphodiesterase activity in screening assays since aggregate formation is known to lead to polypeptide precipitation and to potentially impair activity of a polypeptide. The present invention is directed to the generation of long form PDE⁴ polypeptides with improved physicochemical properties which are particularly useful for the purpose of identifying agonists or antagonists of these enzymes.

The problem of aggregate formation of recombinant long form PDE4 polypeptides was overcome in the present invention by generating a polypeptide comprising a long form PDE4 polypeptide sequence with the exception of the PDE4A polypeptide sequence, with an amino-terminal deletion between the L71 splice site and the UCR1 start said polypeptide exhibiting decreased aggregate formation, as shown in Figures 2 and 3. These polypeptides have a specific activity that is comparable to the specific activity of the native recombinant proteins, as is shown in Figure 1. In a preferred embodiment, the invention provides a polypeptide comprising a long form PDE4 polypeptide sequence, with an amino-terminal deletion wherein the proportion of non-aggregated long form PDE4 in the total protein preparation is from 55 %, preferably from 60%, more preferably from 65%, most perferably from 68% to 100%, preferably to 90 %, more preferably to 80 %, most preferably to 70% of total protein, as determined by the quantitation of eluted fractions from a gel filtration of the long form PDE4 fractions. Quantitation is performed as follows: Equal volume aliquots of fractions from Size Exclusion Chromatography, run as hereinbefore described, are analyzed by SDS-PAGE (one gel per run and isoform, Figure 3). Optical densities of Coomassie stained long form PDE4 are integrated as follows: After electrophoresis the Coomassie stained polyacrylamide gel was imaged by a video imaging system (Figure 1). Optical densities of PDE bands were integrated using a Macintosh computer and the public domain software "NIH Image", version 1.61 (developed at the U.S. National Institutes of Health and available on the Internet at http://rsb.info.nih.gov/nih-image/). The integrated arbitrary units per PDE band as returned by the software reflect the relative PDE concentrations within the original pools. Integrated densities from PDE bands of fractions 2-4 are added up, giving a relative amount of aggregated PDE, PDE_{agg} (Figure 3). These fractions contain molecules of at least 2 Mio Daltons. Similarly, integrated densities from PDE bands of fractions 5-11 are added up, giving a relative amount of non-aggregated PDE, PDE_{non-agg}. In fractions 5-11, molecules in the range of 0.3 Mio Daltons elute. Relative amount of total PDE, PDEₜₒₜₐₗ, is given by the sum of PDE_{agg} and PDE_{non-agg}. The percentage of non-aggregated PDE is given by the ratio of PDE_{non-agg} and PDEₜₒₜₐₗ, multiplied by 100.

In an even more preferred embodiment, the polypeptide sequence is a PDE4D polypeptide sequence, preferably a long form PDE4D polypeptide sequence.

Methods of generating amino-terminal deletion mutants of polypeptides are well known. Such deletion mutants can be generated by using site directed mutagenesis, e.g. by using PCR (Sambrook et al., Molecular Cloning: A Laboratory Manual (1989), Cold Spring Harbor Laboratory Press, New York, USA).

In one embodiment of the present invention, the polypeptide comprises a PDE polypeptide sequence starting between 25 amino acids, preferably between 20 amino acids, more preferably between 15 amino acids, even more perferably between 10 amino acids, most preferably between 5 amino acids upstream of LF1 and the LF1 site.

In another embodiment the PDE long form polypeptide sequence starts at any amino acid located between the LF1 splice site and the first amino acid of the UCR1 start of the native long form PDE4 polypeptide. In the case of the most preferred embodiment of the long form PDE4 polypeptide, PDE4D, these sites are identical in all of the long form PDE4D isoforms.

In another more preferred embodiment of the present invention, the amino-terminal deletion is such that the PDE polypeptide sequence starts at the LF1 splice site of the native PDE polypeptide. This PDE polypeptide sequence is referred to as the "core construct". The core construct spans a sequence of PDE which is shared by all isoforms. The core construct sequence of PDE4D is shown in SEQ ID No. 2.

In another more preferred embodiment of the present invention, the start of the PDE polypeptide sequence is located 13 amino acids upstream of the UCR1 start of the native PDE polypeptide. The location of this position in the long form PDE4D isoforms and the core construct of PDE4D is shown in SEQ ID No. 1-7.

The basis for the generation of the above mentioned long form PDE4 polypeptide with an amino-terminal deletion and improved physicochemical characteristics can be any isoform of long form PDE4, with the exception of PDE4A. For the most perferred embodiment the long form PDE4 polypeptide sequence is an isoform of PDE4D selected from the group consisting of D3, D4, D5, D6, D7, and D8. However, the present invention also extends to further isoforms of long form PDE4D that have not yet been identified.

Also of use to the present invention are mutants of the long form PDE4 polypeptide herein before described. Of particular interest is a long form PDE⁴ polypeptide sequence comprising one or more mutations of Serine residues. Preferably, said Serines are mutated to either Alanine or Aspartic acid. In a most preferred embodiment, said Serine residues are selected from the group consisting of Ser54 and Ser579. Such Serines represent targets for phosphorylation of long form PDE⁴ polypeptides. Phosphorylation of some of these sites has been shown to activate or ihibit the phosphodiesterase activity of PDEs.

Methods of generating amino acid substitutions in polypeptides are well known to those skilled in the art of Molecular Biology, e.g. methods such as site directed mutagenesis as described in Sambrook et al., Molecular Cloning: A Laboratory Manual (1989), Cold Spring Harbor Laboratory Press, New York, USA.

In another preferred embodiment of the present invention, the long form PDE⁴ polypeptide hereinbefore described exhibits decreased tubulin association, thus allowing to obtain purer long form PDE⁴ polypeptide preparations. To determine the decrease in tubulin association, the ratio of PDE molecules per tubulin molecules in the preparation of PDE polypeptide is calculated by quantitation of SDS-PAGE. Preferably, the ratio of PDE molecules per tubulin molecules is at least 5, preferably at least 10, more preferably at least 15, even more preferably at least 20, most preferably at least 25 molecules of PDE per molecules of tubulin. The tubulin content of PDE preparations is determined as follows: Equal volume aliquots of fractions from SEC are analyzed by SDS-PAGE (one gel per run and isoform, Figure 3). Optical densities of Coomassie stained PDE and tubulin bands are integrated as described hereinbefore for PDE aggregation quantitation. Integrated densities from PDE bands of fractions 2-11 are added up, giving a relative amount of total PDE, PDEₜₒₜₐₗ. Likewise, integrated densities from tubulin bands of fractions 2-11 are added up, giving tubulin₂₋₁₁, and thereby the relative amount of total tubulin, tubulinₜₒₜₐₗ. The ratio of PDE and tubulin (Figure 4C) is identical with the ratio of the relative amount of total PDE4D, PDEₜₒₜ, and relative amount of total tubulin, tubulinₜₒₜₐₗ, on a mass per mass basis.

The long form PDE⁴ polypeptides from which the peptide of this invention is generated by amino-terminal deletion as hereinbefore described include polypeptides that are substantially homologous or identical to the polypeptides shown in SEQ ID No. 1-7 and have, in their native form, the phosphodiesterase activity and specificity long form of PDE⁴. In particular, these long form PDE⁴ polypeptides also include the previously published polypeptide sequences of PDE4D3 to D8 as defined in U50159 (D3), L20969 (D4), S:1059276 (D5) and the sequences for D6, D7 and D8 as disclosed in WO02/074992. As used herein, two polypeptides (or a region of the polypeptides) are substantially homologous or identical when the amino acid sequences are at least 45-55%, typically at least 70-75%, more typically at least 80-85%, and most typically greater than 90% or more homologous or identical. To determine the percent homology or identity of two amino acid sequences, the sequences are aligned for optimal comparison purposes (e.g., gaps can be introduced in the sequence of one polypeptide for optimal alignment with the other polypeptide or nucleic acid molecule). The amino acid residues or nucleotides at corresponding amino acid positions are then compared. When a position in one sequence is occupied by the same amino acid residue as the corresponding position in the other sequence, then the molecules are homologous at that position. As used herein, amino acid "homology" is equivalent to amino acid "identity". The percent homology between the two sequences is a function of the number of identical positions shared by the sequences *(i*.*e*., percent homology equals the number of identical positions/total number of positions times 100). The long form PDE⁴ polypeptide sequences of the present invention are preferably mammalian, most preferably human. The present invention also includes variants of the polypeptides hereinbefore described with deletions, insertions, inversions, repeats, and substitutions selected according to general rules known in the art so as to have little effect on activity. For example, guidance concerning how to make phenotypically silent amino acid substitutions is provided in Bowie, Science 247 (1990), 1306-1310, wherein the authors indicate that there are two main strategies for studying the tolerance of an amino acid sequence to change.

The present invention provides polynucleotides that comprise polynucleotide sequences that encode the long form PDE⁴ polypeptide sequences hereinbefore described. As an example for a sequence comprised in such a polynucleotide, the polynucleotide sequence encoding the "core construct" of PDE4D is shown in SEQ ID No. 8. However, the present invention relates to all polynucleotides comprising polynucleotide sequences encoding any of the PDE polypeptide sequences hereinbefore described.

The present invention provides an expression vector or virus comprising a polynucleotide sequence that encodes the long form PDE⁴ polypeptide sequences hereinbefore described and which is capable of directing expression of the polynucleotide sequence in a compatible prokaryotic or eukaryotic host cell. The invention also provides a prokaryotic or eukaryotic host cell transformed or infected with such an expression vector or virus. The invention further provides a process for the production of such a polypeptide comprising culturing the host cell expressing the long form PDE⁴ polypeptide sequences hereinbefore described in a suitable medium so that said polypeptide is expressed, and purifying said polypeptide from the cells. In a preferred embodiment, the virus used for the expression of a modified long form PDE⁴ polypeptide is a recombinant baculovirus. Methods of generating recombinant baculoviruses for expression of recombinant proteins are well known to the man skilled in the art. Preferably, the recombinant baculovirus is based on pFASTBac as the transfer vector. The host cell of a preferred embodiment of the invention is an insect cell. Insect cells that can be infected with recombinant baculoviruses for the purpose of expressing recombinant proteins are well known in the art. The preferred process for the production of a modified long form PDE⁴ polypeptide is a process comprising culturing the insect cell expressing the long form PDE⁴ polypeptide sequences hereinbefore described in a suitable medium so that said polypeptide is expressed, and purifying said polypeptide from the insect cell. To facilitate purification, the long form PDE4 polypeptide may also comprise a tag. Different types of such tags and the methods to join them with polynucleotide sequences encoding a polypeptide are well known to the person skilled in the art, e.g. myc-tag, FLAG-tag, six Histidine tags (hereinafter referred to as 6xHis tag). A review on tagging of proteins can be found e.g. in Fritze and Anderson, Methods in Enzymology (2000) 327, 3-16. It is well known that such tags may be joined to either side of the polypeptide. Some tags are known which may also be removed enzymatically from the purified polypeptide, e.g. FLAG-tag fused to the amino-terminus of a protein may be removed by digestion with bovine enterokinase (Hopp, T.P.(1988) Biotechnology, 6, 1204-1210). In a preferred embodiment the long from PDE4 polypeptides hereinbefore described are linked at their C-terminus to a 6xHis tag. The use of such 6xHis tags for ease of purification of recombinant polypeptides, and methods of purification of 6xHis tagged polypeptides are well known (e.g. in Sisk et al. (1994) J. Virol. 68, 766, and Petty, K.J. (1996) in: Ausubel, F.M. et al., eds. Current Protocols in Molecular Biology, Vol. 2, New York: John Wiley and Sons).

In a preferred embodiment, the polypeptide of the present invention is purified. As used herein, a polypeptide is said to be "purified" when it is substantially free of cellular material when it it isolated from recombinant and non-recombinant cells. A polypeptide, however, can be joined to another polypeptide with which it is not normally associated in a cell (e.g. in a "fusion protein") and still be "purified". Preferably, the polypeptides of the present invention are purified. The polypeptides of the invention can be purified to homogeneity. It is understood, however, that preparations in which the polypeptide is not purified to homogeneity are useful. The critical feature is that the preparation allows for the desired function of the polypeptide, even in the presence of considerable amounts of other components. Thus, the invention encompasses various degrees of purity. In one embodiment, the language "substantially free of cellular material" includes preparations of the polypeptide having less than about 30% (by dry weight) other proteins (i.e., contaminating protein), less than about 20% other proteins, less than about 10% other proteins, or less than about 5% other proteins. When a polypeptide is recombinantly produced, it can also be substantially free of culture medium, i.e., culture medium represents less than about 20%, less than about 10%, or less than about 5% of the volume of the polypeptide preparation.

The present invention also provides for a use of the polypeptides hereinbefore described for identifying an agonist or antagonist of phosphodiesterase activity.

The polypeptides of the present invention can be used for a process of identifying and obtaining a drug candidate for therapy of a vascular disorder, said process comprising measuring the activation or inhibition of the phosphodiesterase activity of any of the above mentioned polypeptides. Different methods for measuring phosphodiesterase activity are known, eg. from Owens et al., Biochem. J. (1997) 326, 53-60. Such a drug candidate may be a compound that is either an agonist or an antagonist of the phosphodiesterase activity of PDE.

The present invention also provides a screening assay for the identification of agonists or antagonists of phosphodiesterase activity comprising at least one of the above described polypeptides. In addition, the polypeptides hereinbefore described can be used for crystallization. Crystals of the polypeptides are useful for assaying the interaction of compounds with phosphodiesterases.

Furthermore, the present invention provides a kit for identifying agonists or antagonists of phosphodiesterase activity comprising at least one of the polypeptides hereinbefore described, and a radioactive or IMKP - FP phosphodiesterase kit.

The invention will be further described by the following non-limiting examples.

### Examples

In the following the term "isoform" relates to the natural PDE4D splice variants D3 and D5-D8 and the invented core variant, DC.

### Cloning of the PDE4D isoforms 3-8 and the core construct

### Core construct:

A cDNA encoding the core fragment that is common for all the PDE4D isoforms 3-8 starting with the amino acid sequence FDV carboxyterminal to the LF1 splice site was generated by PCR using a 5' oligonucleotide with a HindIII cloning site (gatgaattcaagctttttgatgtggacaatggcaca) introducing two additional amino acids (K and L) in front of the FDV sequence. At the 3' end a set of primers was used that either generated the native sequence (gtgatatctcattatcacgtgtcaggagaacgatcatctatgaca) or added a sequence encoding 6xHis residues (gtgatatctcattatcaa tgggatggtgatggtgcgtgtcaggagaacgatcatctatgac) to enable rapid purification of the recombinant proteins. The cDNA encoding the core construct was cloned as a EcoRI-EcoRV fragment into the expression vector pENTR^{™}1a (GIBCO/BRL)
Isoforms (except core construct):
The DNA fragments encoding the isoform specific N-termini were generated by using synthetic oligonucleotides with terminal restriction enzyme sites for EcoRI and HindIII incorporated for directional cloning. These isoform specific DNA fragments were fused to the core construct sequence via the HindIII site introducing two additional amino acid residues (K and L). The integrity of the clones was confirmed by DNA sequencing prior to expression.

### Expression of the PDE4D isoforms and the core construct in insect cells

The cDNAs were cloned into the pFASTBAC1 vector (Life Technologies. Inc) for expression in insect cells and the products were confirmed by sequencing. After recombination into the baculovirus genome the purified viral DNAs were transformed into the insect cells. Sf9 cells were cultured at 27°C in TC100 medium (BioWhittaker) with 5% (v/v) fetal calf serum. Virus stocks were generated with a titer of 1.5x 10⁹ pfu/ml. For large scale production of the isoforms 1-24 L fermentations of Sf9 cells were infected with a MOI of 1.

In one example, the 6xHis tagged PDE4D polypeptides DC, D3, D5, D6, D7 and D8 were produced in Sf9 cells in 1L spinner flasks using SF1 medium in the absence of serum. Infected cells were harvested 3 days after infection with the recombinant baculoviruses.

In another example, the PDE4D core construct DC was produced in a 24 L Airlifter Fermenter with 15 L medium (SF1 with 0% serum), 0.15 L lipids and 9 L Sf9 cells. During the entire fermentation procedure the cells were cultured at pH 6.2, 27.0+/- 0.2°C and a pO₂ of 30.0+/- 0.5 %. Cells were grown for 3 days. The cell number at infection was 2.3x10⁶ cells/ml. Cells were infected with 450 ml recombinant baculovirus. Cells were harvested at 68h post infection and the cell pellet as well as the concentrated supernatant stored at -80°C until further processing.

### Purification of 6xHis tagged PDE4D isoforms

Sf9 cells from 1 liter culture broth, overexpressing the respective isoform, were resuspended on ice in 50 ml 50 mM HEPES pH 7.8, 300 mM NaCl 10 mM imidazole, 1 mM DTT, supplemented with protease inhibitors (one protease inhibitor cocktail tablet "complete, EDTA-free"; Roche). Opening of cells was performed by use of a 50 ml Dounce homogenizator and the homogeneous mixture was centrifuged for 1 hour at 70'000 g and 4°C (Kontron TFT 45.94 rotor at 30'000 rpm). The supernate was filtrated through a filter with a pore size of 1.2 µM (Minisart; Sartorius, Germany) and then applied to a 6 ml Ni-NTA agarose column at 2 ml/min. After equilibration with 50 mM HEPES pH 7.8, 300 mM NaCl, 10 mM imidazole, protein was eluted with a linear 30 ml gradient from 10 to 230 mM imidazole in the same buffer. Fractions containing the PDE4D isoform as analyzed by Coomassie stained SDS-PAGE were pooled and stored frozen at -80°C. Fresh Ni-NTA agarose material was used for every different PDE4D isoform preparation in order to prevent cross contamination of isoforms.

### Specific activities of 6xHis tagged PDE4D isoforms

Relative concentrations of Ni-NTA agarose purified isoform preparations were estimated by SDS-PAGE. Equal volume amounts of isoform preparations were applied to a gradient gel (4-12% NuPage; Invitrogen). After electrophoresis the Coomassie stained polyacrylamide gel was imaged by a video imaging system (Figure 1). Optical densities of PDE4D bands were integrated using a Macintosh computer and the public domain software "NIH Image", version 1.61 (developed at the U.S. National Institutes of Health and available on the Internet at http://rsb.info.nih.gov/nih-image/). The integrated arbitrary units per PDE4D band as returned by the software reflect the relative PDE4D concentrations within the original pools (Figure 1, "amount"). Identities of PDE4D and tubulin bands had been verified by independent SDS-PAGE, excision of corresponding bands, trypsin cleavage and identification of tryptic peptides by MALDI-MS.

Activities of equal volume amounts of 10⁶-fold diluted purified isoforms were determined by use of a commercial radioactive phosphodiesterase assay (cAMP-dependent phosphodiesterase [³H] assay; Amersham Pharmacia Biotech), following the instructions of the manufacturer. The obtained arbitrary activity units reflect the relative PDE4D activities within the original pools (Figure 1, "activity").

Relative specific activities of PDE4D isoforms were calculated by dividing relative activity values by relative concentration values (Figure 1, "specific activity").

### Qualitative investigation of aggregation by size exclusion chromatography (SEC)

50 µl of Ni-NTA agarose purified isoform preparation was injected into a Superose 12 size exclusion column (type PC3.2/30; Amersham Pharmacia Biotech), equilibrated with 50 mM TrisHCl pH 7.7, 100 mM NaCl, 0.5 mM MgCl2 at a flow rate of 0.1 ml/min at 4°C. Chromatograms were recorded at 278 nm. Starting from the elution volume, the column eluate was collected as 50 µl fractions (Figure 2).

### Quantification of aggregates and tubulin content

Equal volume aliquots of fractions from SEC were analyzed by SDS-PAGE (one gel per run and isoform, Figure 3). Optical densities of Coomassie stained PDE4D and tubulin bands were integrated as described above. Integrated densities from PDE4D bands of fractions 2-4 were added up, giving a relative amount of aggregated PDE4D, PDE_{agg} (Figure 3). Similarly, integrated densities from PDE4D bands of fractions 5-11 were added up, giving a relative amount of non-aggregated PDE4D, PDE_{non-agg}. Relative amount of total PDE4D, PDEₜₒₜₐₗ, is given by the sum of PDE_{agg} and PDE_{non-agg}. The percentage of non-aggregated PDE is given by the ratio of PDE_{non-agg} and PDEₜₒₜₐₗ, multiplied by 100 (Figure 4B).

Similarly, integrated densities from tubulin bands of fractions 2-11 were added up, giving tubulin₂₋₁₁, and thereby the relative amount of total tubulin, tubulinₜₒₜₐₗ. The ratio of PDE and tubulin (Figure 4C) is identical with the ratio of relative amount of total PDE4D, PDEₜₒₜ, and relative amount of total tubulin, tubulinₜₒₜₐₗ.

### Activity assay and inhibition of phosphodiesterase activity

An IMAP FP-Assay was used for the determination of phosphodiesterase activity. The phosphodiesterase activity of the core contruct and PDE4D3 was measured using the HEFP Phosphodiesterase Assay Kit (Molecular Devices). 2 µl of PDE4D3 or PDE4D core construct, 2 µl of cAMP (to a final concentration of 40 nM) and 1 µl of test substance or carrier were incubated for 45 min on a shaker. 12 µl of Binding Solution provided by the kit (with beads diluted 1:320) were added, and the reaction mixture incubated on a shaker for 2 hours. Fluorescence polarisation of the samples was measured in a Packard BioScience Fusion a-FP HT using as an emission filter a Polarizer 535, and as an excitation filter, Fluorescein 485/20. Inhibition of the phosphodiesterase activity of the PDE4D core construct was determined using Rolipram as inhibitor, and using PDE4D core construct at 30 ng/ml.

## Claims

1. A polypeptide comprising a long form PDE4 polypeptide sequence with an amino-terminal deletion, wherein the polypeptide sequence starts at any amino acid located between the LF1 splice site and the first amino acid of the UCR1 start of the native long form PDE4 polypeptide, said polypeptide exhibiting decreased aggregate formation.

2. The polypeptide of claim 1 wherein said PDE4 polypeptide sequence is a PDE4D polypeptide sequence.

3. The polypeptide of any of claims 1 to 2, wherein the polypeptide sequence starts at the LF1 splice site of the native PDE4 polypeptide.

4. The polypeptide of any of claims 1 to 3, wherein the PDE4 polypeptide sequence start is located 13 amino acids upstream of the UCR1 start of the native PDE4 polypeptide.

5. The polypeptide of any of claims 2 to 4 wherein the PDE4D polypeptide sequence is a sequence of an isoform selected from the group consisting of D3, D4, D5, D6, D7, and D8.

6. The polypeptide of claims 1 to 5 wherein said polypeptide comprises one or more mutations of Serine residues.

7. The polypeptide of claim 6 wherein said Serine residues are mutated to either Alanine or Aspartic acid.

8. The polypeptide of claims 6 or 7 wherein said Serine residues are selected from the group consisting of Ser residues corresponding to Ser54 and Ser579 in Seq ID No. 1.

9. The polypeptide of any one of claims 1 to 5 wherein said polypeptide exhibits decreased tubulin association.

10. A polynucleotide sequence encoding a polypeptide as claimed in claims 1 to 9.

11. An expression vector or virus comprising a polynucleotide sequence as claimed in claim 10 and being capable of directing expression of the polynucleotide sequence in a compatible prokaryotic or eukaryotic host cell.

12. A virus as claimed in claim 11 wherein the virus is a recombinant baculovirus.

13. A prokaryotic or eukaryotic host cell transformed or infected with the expression vector or virus of claims 11 or 12.

14. A host cell as claimed in claim 13, wherein the host cell is an insect cell.

15. A process for the production of a polypeptide as claimed in claims 1 to 9, comprising culturing the host cell of claims 13 or 14 in a suitable medium so that said polypeptide is expressed, and purifying said polypeptide from the cells.

16. A process for the production of a polypeptide as claimed in claim 15 wherein the host cell is an insect cell.

17. A polypeptide according to claims 1 to 9 produced by the process of claims 15 or 16.

18. A screening assay for the identification of agonists or antagonists of phosphodiesterase activity comprising at least one of the polypeptides of claims 1 to 9.

19. A process for identifying a drug candidate for therapy of an inflammatory disease, said process comprising measuring the activation or inhibition of the phosphodiesterase activity of any of the polypeptides claimed in claims 1 to 9.

20. Use of a polypeptide of claims 1 to 9 for identifying an agonist or antagonist of phosphodiesterase activity.

21. Use of a polypeptide of claims 1 to 9 for crystallization.

22. A kit for identifying agonists or antagonists of phosphodiesterase activity comprising at least one of the polypeptides of claims 1 to 9 and a radioactive or IMAP-FP phosphodiesterase kit.

## Patentansprüche

1. Polypeptid, umfassend eine Sequenz der langen Form des PDE4-Polypeptids mit einer aminoterminalen Deletion, wobei die Polypeptidsequenz bei einer beliebigen Aminosäure beginnt, die zwischen der LF1-Spleißstelle und der ersten Aminosäure des UCR1-Startpunktes der nativen langen Form des PDE4-Polypeptids liegt, wobei das Polypeptid erniedrigte Aggregatbildung aufweist.

2. Polypeptid nach Anspruch 1, wobei die PDE4-Polypeptidsequenz eine PDE4D-Polypeptidsequenz ist.

3. Polypeptid nach einem der Ansprüche 1 bis 2, wobei die Polypeptidsequenz an der LF1-Spleißstelle des nativen PDE4-Polypeptids beginnt.

4. Polypeptid nach einem der Ansprüche 1 bis 3, wobei sich der Startpunkt der PDE4-Polypeptidsequenz 13 Aminosäuren stromaufwärts des UCR1-Startpunkts des nativen PDE4-Polypeptids befindet.

5. Polypeptid nach einem der Ansprüche 2 bis 4, wobei die PDE4D-Polypeptidsequenz die Sequenz einer Isoform ist, ausgewählt aus der Gruppe bestehend aus D3, D4, D5, D6, D7 und D8.

6. Polypeptid nach den Ansprüchen 1 bis 5, wobei das Polypeptid eine oder mehrere Mutationen von Serinresten umfasst.

7. Polypeptid nach Anspruch 6, wobei die Serinreste entweder zu Alanin oder Asparaginsäure mutiert sind.

8. Polypeptid nach den Ansprüchen 6 oder 7, wobei die Serinreste ausgewählt sind aus der Gruppe bestehend aus Serinresten, die Ser 54 und Ser 579 in Seq ID NO. 1 entsprechen.

9. Polypeptid nach einem der Ansprüche 1 bis 5, wobei das Polypeptid erniedrigte Tubulinassoziation aufweist.

10. Polynucleotidsequenz, die ein Polypeptid nach den Ansprüchen 1 bis 9 codiert.

11. Expressionsvektor oder Virus, umfassend eine Polynucleotidsequenz nach Anspruch 10 und fähig, die Expression der Polynucleotidsequenz in einer kompatiblen prokaryontischen oder eukaryontischen Wirtszelle zu steuern.

12. Virus nach Anspruch 11, wobei das Virus ein rekombinantes Baculovirus ist.

13. Prokaryontische oder eukaryontische Wirtszelle, die mit dem Expressionsvektor oder Virus nach Anspruch 11 oder 12 transformiert oder infiziert ist.

14. Wirtszelle nach Anspruch 13, wobei die Wirtszelle eine Insektenzelle ist.

15. Verfahren für die Produktion eines Polypeptids nach den Ansprüchen 1 bis 9, umfassend die Züchtung der Wirtszelle nach den Ansprüchen 13 oder 14 in einem geeigneten Medium, so dass das Polypeptid exprimiert wird, und die Reinigung des Polypeptids aus den Zellen.

16. Verfahren für die Produktion eines Polypeptids nach Anspruch 15, wobei die Wirtszelle eine Insektenzelle ist.

17. Polypeptid nach den Ansprüchen 1 bis 9, das durch das Verfahren nach den Ansprüchen 15 oder 16 produziert wurde.

18. Screening-Assay zur Identifizierung von Agonisten oder Antagonisten von Phosphodiesteraseaktivität, umfassend mindestens eines der Polypeptide nach den Ansprüchen 1 bis 9.

19. Verfahren zur Identifizierung eines Wirkstoffkandidaten zur Therapie einer entzündlichen Erkrankung, umfassend die Messung der Aktivierung oder Hemmung der Phosphodiesteraseaktivität eines der Polypeptide nach einem der Ansprüchen 1 bis 9.

20. Verwendung eines Polypeptids nach einem der Ansprüchen 1 bis 9 zur Identifizierung eines Agonisten oder Antagonisten von Phosphodiesteraseaktivität.

21. Verwendung eines Polypeptids nach den Ansprüchen 1 bis 9 zur Kristallisation.

22. Kit zur Identifizierung von Agonisten oder Antagonisten von Phosphodiesteraseaktivität, umfassend mindestens eines der Polypeptide nach den Ansprüchen 1 bis 9 und einen radioaktiven oder IMAP-FP-Phosphodiesterase-Kit.

## Revendications

1. Polypeptide comprenant une séquence polypeptidique d'une PDE4 sous forme longue avec une délétion amino-terminale, où la séquence polypeptidique commence à un aminoacide quelconque situé entre le site d'épissage LF1 et le premier aminoacide constituant le début du domaine UCR1 du polypeptide PDE4 sous forme longue natif, ledit polypeptide présentant une quantité d'agrégats diminuée.

2. Polypeptide selon la revendication 1 ladite séquence polypeptidique de PDE4 étant une séquence polypeptidique de PDE4D.

3. Polypeptide selon l'une quelconque des revendications 1 à 2, la séquence polypeptidique commençant au site d'épissage LF1 du polypeptide PDE4 natif.

4. Polypeptide selon l'une quelconque des revendications 1 à 3, le début de la séquence polypeptidique de PDE4 étant localisé 13 aminoacides en amont du début du domaine UCR1 du polypeptide PDE4 natif.

5. Polypeptide selon l'une quelconque des revendications 2 à 4, la séquence polypeptidique de PDE4D étant une séquence d'une isoforme choisie dans le groupe constitué par D3, D4, D5, D6, D7 et D8.

6. Polypeptide selon les revendications 1 à 5, ledit polypeptide comprenant une ou plusieurs mutations de résidus de sérine.

7. Polypeptide selon la revendication 6, dans lequel lesdits résidus de sérine sont mutés en alanine ou en acide aspartique.

8. Polypeptide selon les revendications 6 ou 7, dans lequel lesdits résidus de sérine sont choisis dans le groupe constitué par les résidus de sérine correspondant à Ser54 et Ser579 dans SEQ ID n° : 1.

9. Polypeptide selon l'une quelconque des revendications 1 à 5, ledit polypeptide présentant une association à la tubuline diminuée.

10. Séquence de polynucléotide codant pour un polypeptide selon les revendicatons 1 à 9.

11. Vecteur d'expression ou virus comprenant une séquence de polynucléotide selon la revendication 10 et capable de diriger l'expression de la séquence de polynucléotide dans une cellule hôte procaryote ou eucaryote compatible.

12. Virus selon la revendication 11, le virus étant un baculovirus recombiné.

13. Cellule hôte procaryote ou eucaryote transformée ou infectée par le vecteur d'expresion ou le virus des revendications 11 ou 12.

14. Cellule hôte selon la revendication 13, la cellule hôte étant une cellule d'insecte.

15. Procédé pour la production d'un polypeptide selon les revendications 1 à 9 comprenant la culture de la cellule hôte des revendications 13 ou 14 dans un milieu approprié de telle sorte que ledit polypeptide soit exprimé, et la purification dudit polypeptide à partir des cellules.

16. Procéde pour la production d'un polypeptide selon la revendication 15, dans lequel la cellule hôte est une cellule d'insecte.

17. Polypeptide selon les revendications 1 à 9, produit par le procédé selon les revendications 15 ou 16.

18. Test de criblage pour l'identification d'agonistes ou d'antagonistes de l'activité phosphodiestérase comprenant au moins un des polypeptides selon les revendications 1 à 9.

19. Procédé pour l'identification d'un médicament candidat pour la thérapie d'une maladie inflammatoire, ledit procédé comprenant la mesure de l'activation ou de l'inhibition de l'activité phoshodiestérase d'un quelconque des peptides selon les revendications 1 à 9.

20. Utilisation d'un polypetide selon les revendications 1 à 9, pour identifier un agoniste ou un antagoiste de l'activité phosphodiestérase.

21. Utilisation d'un polypeptide selon les revendications 1 à 9 pour la cristallisation.

22. Trousse pour l'identification d'agonistes ou d'antagonistes de l'activité phoshodiestérase comprenant au moins un des polypeptides selon les revendications 1 à 9, et un kit de test de phosphodiestérase radioactif ou IMPP-FP.
